# EUROPEAN PATENT SPECIFICATION

(11) **EP 3 672 580 B1**
(45) Date of publication and mention of the grant of the patent: **01.09.2021**
(21) Application number: 18768973.2
(22) Date of filing: 26.07.2018
(51) Int. Cl.: A61K 31/07, A61K 31/202, A61P 25/28, A23L 33/12, A23L 33/10, A23L 33/105

(54) **COMPOSITION COMPRISING A XANTOPHYLL AND AN OMEGA- 3 FATTY ACID AND ITS USE FOR IMPROVING COGNITIVE FUNCTION**
ZUSAMMENSETZUNG MIT EINEM XANTOPHYLL UND EINER OMEGA-3-FETTSÄURE UND DEREN VERWENDUNG ZUR VERBESSERUNG DER KOGNITIVEN FUNKTION
COMPOSITION COMPRENANT UNE XANTOPHYLLE ET UN ACIDE GRAS OMÉGA-3 ET SON UTILISATION POUR L'AMÉLIORIATION DE LA FONCTION COGNITIVE

(30) Priority: 23.08.2017 NL 2019436
(43) Date of publication of application: 01.07.2020
(73) Proprietor: Newtricious B.V., 5223 DE 's Hertogenbosch (NL)
(72) Inventor: JOHNSON, Elizabeth Judith, Norwood, Massachusetts 02062 (US); VAN DER MADE, Sanne Maria, 5258 BZ Berlicum (NL); BIVERT, Marcel Alexander, 6211 JH Maastricht (NL)
(74) Representative: Nederlandsch Octrooibureau
(86) International application number: PCT/NL2018/050522
(87) International publication number: WO 2019/039936

(56) References cited:
- WO-A1-2009/078716
- WO-A1-2016/086157
- WO-A2-2006/116755
- US-A1- 2016 067 203
- JOHNSON ELIZABETH J ET AL: "Cognitive findings of an exploratory trial of docosahexaenoic acid and lutein supplementation in older women", NUTRITIONAL NEUROSCI, MANEY, UK, vol. 11, no. 2, 1 April 2008 (2008-04-01), pages 75-83, XP009188657, ISSN: 1476-8305
- EMILY Y. CHEW ET AL: "Effect of Omega-3 Fatty Acids, Lutein/Zeaxanthin, or Other Nutrient Supplementation on Cognitive Function : The AREDS2 Randomized Clinical Trial", JAMA THE JOURNAL OF THE AMERICAN MEDICAL ASSOCIATION, vol. 314, no. 8, 25 August 2015 (2015-08-25), page 791, XP055459172, US ISSN: 0098-7484, DOI: 10.1001/jama.2015.9677

## Description

### FIELD OF THE INVENTION

The present invention relates to the field of food products, especially food products specifically designed for providing health benefits beyond the basic function of nutrition for a subject consuming the product on a regular basis, *e.g.* on a daily basis. In particular, the invention relates to a non-therapeutic method of amelioration of a cognitive function in a subject, the method comprising administering to the subject a composition comprising: (1) at least one omega-3 fatty acid; and (2) lutein and zeaxanthin, wherein the lutein is dosed to provide an amount of between 0,5 mg and 5 mg of lutein per day and the zeaxanthin is dosed to provide an amount of between 0,15 mg and 0,40 mg of zeaxanthin per day. In addition, the invention particularly relates to a composition comprising at least one omega-3 fatty and lutein and zeaxanthin, for use in the prevention or the treatment of the decline of a cognitive function of a subject or for use in the amelioration of a cognitive function of a subject, wherein the lutein is dosed to provide an amount of between 0,5 mg and 5 mg of lutein per day and the zeaxanthin is dosed to provide an amount of between 0,15 mg and 0,40 mg of zeaxanthin per day. Particular suitable omega-3 fatty acid is docosahexaenoic acid.

### BACKGROUND OF THE INVENTION

A decline in cognitive (thinking) function seen in, mainly elderly, healthy human subjects is considered to be a normal consequence of aging. However, nowadays, more and more attention is devoted to decline in cognitive function of otherwise healthy subjects, since such decline can be an early sign of the risk for developing mild cognitive impairment (MCI), and, subsequently, even for developing dementia. With a world-wide population comprising an ever increasing number of elderly subjects, there is thus a high and urgent need in the art for methods of treating a decline in memory and cognitive function and of preserving cognitive function. The more since even the mildest decline in cognitive function may result in the affected (elderly) subjects suffering from deterioration in memory and learning, attention and concentration, use of language, and other mental functions, thereby having a large impact on their quality of life. Methods of treating a decline in memory and cognitive function and of preserving cognitive function of subjects, *e.g*. otherwise healthy subjects such as elderly subjects, is therefore urgently needed in the art. The more since about 5% to 15% of subjects who develop MCI, subsequently develop a, often highly debilitating, dementia within a year, making MCI a strong risk factor for developing dementia such as Alzheimer's disease within a short period of time.

International patent application PCT/US2006/016506 with publication number WO 2006/116755, describes compositions and methods for increasing the absorption of dietary carotenoids in humans. Such compositions and methods can be used to slow, reduce, or prevent the onset of neurological or memory disorders, to improve learning and cognition, and to improve memory retention and acquisition. PCT/US2006/016506 discloses pharmaceutical compositions comprising a therapeutically or prophylactically effective amount of a lutein and DHA.

Johnson et al., in Nutritional Neuroscience, 2008, Vol. 11, No. 2, pp.75-83, describe cognitive findings of an exploratory trial of DHA and lutein supplementation in older women. The cognitive benefit of DHA and lutein in unimpaired elder women was explored in the context of a 4-month, double-blind, intervention trial of DHA and lutein supplementation for eye health. Subjects underwent cognitive tests measuring verbal fluency, memory, processing speed and accuracy, and self-reports of mood at randomization and upon completion of the trial.

International patent application PCT/US2015/062721 with publication number WO 2016/086157, describes infant formula comprising human milk oligosaccharides, polyunsaturated fatty acids such as DHA, nucleotides, and lutein. Furthermore, methods of use of the infant formulas are provided in PCT/US2015/062721 for improving at least one of cognition, learning, and memory in an infant. The methods include administering the infant formulas to an infant.

US patent application US 14/787,399 with publication number US2016/0067203, describes the provision of an internal composition capable of exerting various physiological functions such as a brain-function improving function, the internal composition including DHA, capsanthin, lutein, and zeaxanthin as active ingredients.

International patent application PCT/NL2008/050810 with publication number WO 2009/078716, describes food products specifically designed for providing health benefits beyond the basic function of nutrition for a subject consuming the product on a regular basis, e.g. on a daily basis. Production of eggs is described, in particular chicken eggs, which are enriched in one or more pharmacologically active nutrients, such as omega-3 fatty acids, especially DHA, xantophylls, especially lutein and/or zeaxanthin. The egg yolk obtained from the aforementioned eggs can suitably be used in high amounts as a component of a functional food product having all the characteristics that are normally required for such functional food products, such functional food products for example being intended for reducing the incidence and/or severity of age-related macular degeneration, cataract, atherosclerosis and/or for improving skin health.

Chew et al., in JAMA, 2015, 25 August, Vol. 314, Issue 8, pp.791-801, describe results of the Age-Related Eye Disease Study 2 (AREDS2) randomized clinical trial, relating to the effect of amongst others omega-3 fatty acids and lutein/zeaxanthin on cognitive function. Intake of long-chain polyunsaturated fatty acids (1 g), especially DHA, and/or lutein (10 mg)/zeaxanthin (2 mg) versus placebo were tested in participants who were at risk for developing late age-related macular degeneration. All participants were also given varying combination of vitamin C, vitamin E, beta-carotene, and zinc, known as the Age-Related Eye Disease Study supplement. Several validated cognitive function tests were administered via telephone at baseline and every two years during the five year study.

### SUMMARY OF THE INVENTION

This invention provides compositions that conserve or maintain or increase or enhance or ameliorate the cognitive function in a subject, preferably a healthy subject, such as for example a composition that prevents the loss of cognitive function during the course of time. In addition, this invention provides compositions that treat, prevent, delay or slow down the loss of cognitive function in a subject, preferably a healthy subject, such that the development or occurrence of mild cognitive impairment (MCI) or a dementia such as Alzheimer's disease is prevented, or such that the onset of said impairment or disorder, *e.g*. dementia, is delayed.

A first aspect of the invention relates to a non-therapeutic method of amelioration of a cognitive function in a subject, the method comprising administering to the subject a composition comprising: (1) at least one omega-3 fatty acid; and (2) lutein and zeaxanthin, wherein the lutein is dosed to provide an amount of between 0,5 mg and 5 mg of lutein per day and the zeaxanthin is dosed to provide an amount of between 0,15 mg and 0,40 mg of zeaxanthin per day. In an embodiment, in the non-therapeutic method according to the invention, the at least one omega-3 fatty acid is selected from the group consisting of docosahexaenoic acid and eicosapentaenoic acid, preferably the at least one omega-3 fatty acid is docosahexaenoic acid.

In an embodiment, in the non-therapeutic method according to the invention, the subject to whom the composition is administered is a healthy subject, preferably a human subject.

A second aspect of the invention relates to a composition comprising at least one omega-3 fatty acid and lutein and zeaxanthin, for use in the prevention or the treatment of the decline of a cognitive function of a subject or for use in the amelioration of a cognitive function of a subject, wherein the lutein is dosed to provide an amount of between 0,5 mg and 5 mg of lutein per day and the zeaxanthin is dosed to provide an amount of between 0,15 mg and 0,40 mg of zeaxanthin per day. In an embodiment, in the composition for use in the prevention or the treatment of the decline of a cognitive function or for use in the amelioration of a cognitive function according to the invention, the omega-3 fatty acid is selected from the group consisting of docosahexaenoic acid and eicosapentaenoic acid, preferably the omega-3 fatty acid is docosahexaenoic acid.

Also disclosed is a non-therapeutic method of improving a cognitive function in a subject, the method comprising administering to the subject a composition comprising:
(1) at least one omega-3 fatty acid; and
(2) at least one xanthophyll selected from lutein and zeaxanthin, wherein the lutein is dosed to provide an amount of between about 0,2 mg and 15 mg of lutein per day and/or the zeaxanthin is dosed to provide an amount of between about 0,1 mg and 0,6 mg of zeaxanthin per day, and wherein the improvement of said cognitive function is the prevention or the treatment of the decline of a cognitive function or the amelioration of a cognitive function.

Also disclosed is a composition comprising at least one omega-3 fatty acid and at least one xanthophyll selected from lutein and zeaxanthin, for use in the prevention or the treatment of the decline of a cognitive function of a subject or for use in the amelioration of a cognitive function of a subject, wherein the lutein is dosed to provide an amount of between about 0,2 mg and 15 mg of lutein per day and/or the zeaxanthin is dosed to provide an amount of between about 0,1 mg and 0,6 mg of zeaxanthin per day.

In particular, the at least one xanthophyll are the two xanthophylls lutein and zeaxanthin, according to the invention.

Typically, for the non-therapeutic method of the invention and/or for the composition for use in the prevention or the treatment of the decline of a cognitive function of a subject or for use in the amelioration of a cognitive function of a subject according to the invention, the at least one omega-3 fatty acid is dosed to provide an amount of between about 50 mg and 3 g of omega-3 fatty acid per day, preferably at least about 60 mg, more preferably at least about 75 mg, most preferably between about 120 mg and 600 mg.

It is preferred that for the non-therapeutic method of the invention and/or for the composition for use in the prevention or the treatment of the decline of a cognitive function of a subject or for use in the amelioration of a cognitive function of a subject according to the invention, the lutein is dosed to provide an amount of between 1,1 mg and 1,8 mg lutein per day and the zeaxanthin is dosed to provide an amount of between 0,17 mg and 0,30 mg zeaxanthin per day.

In an embodiment, the composition for use in the prevention or the treatment of the decline of a cognitive function or for use in the amelioration of a cognitive function according to the invention is administered to a subject, preferably a human subject, wherein the subject is preferably a healthy subject.

The composition may be formulated as a pharmaceutical composition or as a nutritional supplement, or the composition may be present within an enriched foodstuff, such as a drink.

In an embodiment, with the non-therapeutic method according to the invention, the cognitive function that improves when the composition is administered to a subject, is any one or more of the group consisting of general alertness, motor speed, short-term memory such as short-term visual recognition memory, working memory, visual memory, new learning, sustained attention, and planning efficiency.

In an embodiment, with the composition for use in the prevention or the treatment of the decline of a cognitive function or for use in the amelioration of a cognitive function according to the invention, the cognitive function that improves when the composition is administered to a subject, is any one or more of the group consisting of general alertness, motor speed, short-term memory such as short-term visual recognition memory, working memory, visual memory, new learning, sustained attention, and planning efficiency.

### DETAILED DESCRIPTION OF THE INVENTION

The present invention will be described with respect to particular embodiments and with reference to certain examples but the invention is not limited thereto but only by the claims.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a composition comprising A and B" should not be limited to compositions consisting only of components A and B, rather with respect to the present invention, the only enumerated components of the composition are A and B, and further the claim should be interpreted as including equivalents of those components, such as derivatives thereof.

The term "improving" in "improving a cognitive function" has its general meaning, and here refers to the prevention of the decline of a cognitive function or the treatment of the decline of a cognitive function or the amelioration of a cognitive function, or the reversal or the restoration of earlier occurred decline of a cognitive function or the delay, postponement or the slowing down of the decline of a cognitive function.

The term "cognitive function" has its general meaning, and at least encompasses the functions of general alertness, motor speed, short-term memory such as short-term visual recognition memory, working memory, visual memory, new learning, sustained attention, and planning efficiency.

The term "cognition" has its regular scientific meaning and here refers to the mental action or process of acquiring knowledge and understanding through thought, experience, and the senses. Cognition (thinking) consists of six main domains: learning and memory, social functioning, language, visuospatial function, complex attention and executive functioning.

The term "basic cognitive functions" has its regular scientific meaning and here refers to:
A. Attention
   a. Selective attention refers to the ability to attend to some stimuli while disregarding others that are irrelevant to the task at hand
      - in general older adults appear to be slower than younger adults in responding to the targets, but are not differentially affected by distraction
   b. Divided attention and attention switching: requires the processing of two or more sources of information or the performance of two or more tasks at the same time
      - Has been associated with age-related declines in performance, particularly when tasks are complex
      - Older adults are more affected by the division of attention than young adults
   c. Sustained attention refers to the ability to maintain concentration on a task over an extended period of time
B. Working Memory
   - Older adults exhibit significant deficits in tasks that involve active manipulation, reorganization, or integration of the contents of working memory.
C. Long-Term memory: Requires retrieval of information that is no longer present or being maintained in an active state
   a. Episodic memory: personally experienced events that occurred in a particular place and at a particular time
      - Seems the most susceptible to brain damage and the most affected by normal ageing
   b. Semantic memory: refers to one's store of general knowledge, including factual information and knowledge of words and concepts
   c. Autobiographical memory involves memory for one's personal past and includes memories that are both episodic and semantic in nature
   d. Procedural memory refers to the knowledge of skills and procedures such as riding a bicycle, playing the piano or reading a book.
   e. Implicit memory refers to a change in behavior that occurs as a result of prior experience, although one had no conscious or explicit recollection of that prior experience
   f. Prospective memory: remembering to do something in the future
D. Perception
   - Reduced in most older adults and not always correctable by external aids

The term "higher-level cognitive functions" has its regular scientific meaning and here refers to:
A. Speech and language
   - Largely intact in older adults under normal conditions, although processing time may be somewhat slower than in young adults
B. Decision making
C. Executive control: multi-component construct that consists of a range of different processes that are involved in the planning, organization, coordination, implementation, and evaluation of many of non-routine activities

[References: Glisky, E. (2007) Changes in Cognitive Function in Human Aging. In: Brain Aging: Models, Methods, and Mechanisms (D. Riddle, editor). Boca Raton (FL): CRC Press / Taylor & Francis; Sanford AM (2017) Mild Cognitive Impairment. Clinics in geriatric medicine 33, 325-337; American Psychiatric Association (2013) Diagnostic and statistical manual of mental disorders. 5th ed. Arlington (VA): American Psychiatric Association; Sachdev PS, Blacker D, Blazer DG et al. (2014) Classifying neurocognitive disorders: the DSM-5 approach. Nature reviews Neurology 10, 634-642]

The term "mild cognitive impairment", or abbreviated to "MCI", has its regular scientific meaning and here refers to impairment in cognition above that which is seen with normal age-related cognitive decline, but not severe enough to cause significantly impaired daily function. MCI generally refers to a decline in the ability to learn new information or recall restored information. It is characterized by the following points:
- Preserved daily functioning
- Impairment in one or more cognitive domains
- Self- or informant-reported cognitive decline
- Does not meet criteria for dementia

The Diagnostic and Statistical Manual of Mental Disorders, 5th Edition (DSM-V) [Reference: American Psychiatric Association, DSM-5 Task Force. (2013) Diagnostic and statistical manual of mental disorders: DSM-5. 5th ed. Washington, D.C.: American Psychiatric Association] classifies MCI as a 'mild neurocognitive disorder', and specifies that there must be both a subjective and objective decline from previous level of functioning in one or more of the six cognitive domains (learning and memory, social functioning, language, visuospatial function, complex attention and executive functioning), which does not substantially interfere with instrumental activities of daily living, and does not occur in the context of delirium or other psychological disorders. MCI exceeds the age-related decline in cognition that is regularly observed as individuals get older. MCI is no precursor to dementia in all cases, nor is it progressive in all cases. Other medical disorders that are positively associated with MCI include Parkinson Disease, traumatic brain injury, cerebrovascular accident, Huntington disease and human immunodeficiency virus.

The term "cognitive decline" has its regular scientific meaning and here also refers to 'forgetfulness' that is consistent with normal ageing. The basic cognitive functions most affected by age are attention and memory. Perception also shows significant age-related declines, which are mainly attributed to declining sensory capacities. In addition, higher-level cognitive functions such as language processing and decision-making may be affected by age.

The term "healthy" in *e.g.* a "healthy subject" of whom a cognitive function is improved according to the invention, has its general meaning, and here also refers to a subject who does notably not suffer from any illnesses or health complaints that influences or impairs cognitive function.

The term "memory disorder" has its regular scientific meaning, and here refers to the result of damage to neuroanatomical structures that hinders the storage, retention and recollection of memories.

The term "cognitive impairment" such as used in "mild cognitive impairment" has its regular scientific meaning, and here refers to any characteristic that acts as a barrier to the cognition process, such as neuropsychological deficits (such as in attention, working memory or executive function).

The term "cognitive disorder" has its regular scientific meaning, and here refers to mental health disorders that primarily affect learning, memory, perception, and problem solving, and include amnesia, dementia, and delirium.

The term "neurological disorder" has its regular scientific meaning, and here refers to any disorder of the nervous system, including neurodegenerative diseases such as dementia and Alzheimer's disease.

As used herein, the term "a xanthophyll" encompasses one or more species of xanthophylls and is equivalent to the term "at least one xanthophyll". Examples of suitable xanthophylls are lutein, meso-zeaxanthin and zeaxanthin.

As used herein, the term "an omega-3 fatty acid" encompasses one or more species of omega-3 fatty acids and is equivalent to the term "at least one omega-3 fatty acid".

As used herein, the term "may" encompasses the word "can," and the term "may be" encompasses the words "is" or "are," depending on context. Furthermore, presence of the word "may" is intended to explain options for practicing or implementing the disclosure, without limitation.

There is ample evidence that xanthophylls have a beneficial effect on inflammatory processes in brain, skin, eye and liver. Xanthophylls are conveniently administered to a subject in need of such a treatment. Lutein, zeaxanthin and omega-3 fatty acids such as docosahexanoic acid (DHA) have antioxidant activity and anti-inflammatory activity, respectively.

United States patent US6,509,178 discloses that DHA is known for improving memory learning and is considered useful for treating *inter alia* Alzheimer's disease.

Disclosed is a non-therapeutic method of improving a cognitive function in a subject, the method comprising administering to the subject a composition comprising:
(1) at least one omega-3 fatty acid; and
(2) at least one xanthophyll,
wherein the improvement of said cognitive function is the prevention or the treatment of the decline of a cognitive function or the amelioration of a cognitive function.

Also disclosed is a composition comprising at least one omega-3 fatty acid and at least one xanthophyll, for use in the prevention or the treatment of the decline of a cognitive function of a subject or for use in the amelioration of a cognitive function of a subject.

Also disclosed is the use of a composition comprising at least one omega-3 fatty acid and at least one xanthophyll for the manufacture of a medicament, wherein the medicament is for the prevention or the treatment of the decline of a cognitive function of a subject or for the amelioration of a cognitive function of a subject.

Also disclosed is a method of treatment of the decline of a cognitive function or of the amelioration of a cognitive function, wherein a composition comprising at least one omega-3 fatty acid and at least one xanthophyll is administered to a subject in need thereof.

Also disclosed is the use of the composition comprising at least one omega-3 fatty acid and at least one xanthophyll of the invention for the prevention or the treatment of the decline of a cognitive function of a subject or for the amelioration of a cognitive function of a subject.

Also disclosed is a method of treating a subject suffering from decline of a cognitive function or at risk for decline of a cognitive function or a subject in need of the amelioration of a cognitive function, comprising administering a composition comprising at least one omega-3 fatty acid and at least one xanthophyll according to the invention to the subject.

Also disclosed is the administration to a subject of a composition comprising at least one omega-3 fatty acid and at least one xanthophyll according to the invention for treating or preventing decline of a cognitive function and/or for amelioration of a cognitive function.

Also disclosed is a composition comprising at least one omega-3 fatty acid and at least one xanthophyll according to the invention for treating or preventing decline of a cognitive function and/or for amelioration of a cognitive function.

In one embodiment, in the non-therapeutic method according to the invention or with regard to the composition for use according to the invention, the cognitive function of the subject to whom the composition is administered was previously declined at the start of administering the composition to said subject.

In one disclosure, in the composition applied in the non-therapeutic method according to the invention or in the composition for use according to the invention, the at least one xanthophyll is selected from the group consisting of lutein, meso-zeaxanthin and zeaxanthin, preferably the selected xanthophyll comprises lutein and zeaxanthin.

In another disclosure, the at least one xanthophyll is selected from antheraxanthin, neoxanthin, violaxanthin, flavoxanthin, α- cryptoxanthin, β-cryptoxanthin, lutein, meso-zeaxanthin and zeaxanthin. In a preferred embodiment, the at least one xanthophyll is selected from the group consisting of lutein and zeaxanthin, preferably the selected xanthophyll comprises lutein and zeaxanthin, according to the invention. Thus, a disclosed embodiment is the composition for use in the non-therapeutic method or for use in the prevention or the treatment of the decline of a cognitive function or for use in the amelioration of a cognitive function, wherein the composition comprises the xanthophylls lutein, meso-zeaxanthin and zeaxanthin, more preferably lutein and zeaxanthin.

In one embodiment, in the composition applied in the non-therapeutic method according to the invention or in the composition for use according to the invention, the at least one omega-3 fatty acid is selected from the group consisting of docosahexaenoic acid and eicosapentaenoic acid, preferably the omega-3 fatty acid is docosahexaenoic acid.

In one embodiment, the composition applied in the method of the invention or the composition for use in the prevention or the treatment of the decline of a cognitive function or for use in the amelioration of a cognitive function according to the invention, further comprises a pharmacologically active compound selected from the group consisting of choline (also referred to as vitamin B4), vitamin D, uridin, folic acid, vitamin E, iodine, selenium and zinc. Particularly preferred is a composition applied in the method of the invention or a composition for use in the prevention or the treatment of the decline of a cognitive function or for use in the amelioration of a cognitive function according to the invention, further comprising the pharmacologically active compound choline. Choline in *e.g.* a food stuff, dietary product, food, beverage, drink, *etc.,* which is frequently administered to a (human) subject such as administered daily, is known to have a beneficial effect on cognition. A suitable source of choline is an egg yolk. Typically, an egg yolk comprises about 150 mg choline.

Thus, the composition applied in the non-therapeutic method of the invention or the composition for use in the prevention or the treatment of the decline of a cognitive function or for use in the amelioration of a cognitive function according to the invention may further comprise an additional pharmacologically active or nutritionally beneficial compound, such as a compound selected from the group consisting of choline, uridin, vitamin D, folic acid, vitamin E, iodine, selenium and zinc. Preferably, said composition comprises choline, or a derivative thereof.

Disclosed is a non-therapeutic method of improving a cognitive function in a subject, the method comprising administering to the subject a composition comprising:
(1) at least one omega-3 fatty acid; and
(2) at least one xanthophyll selected from lutein and zeaxanthin, wherein the lutein is dosed to provide an amount of between about 0,2 mg and 15 mg of lutein per day and/or the zeaxanthin is dosed to provide an amount of between about 0,1 mg and 0,6 mg of zeaxanthin per day, and wherein the improvement of said cognitive function is the prevention or the treatment of the decline of a cognitive function or the amelioration of a cognitive function.

Also disclosed is a composition comprising at least one omega-3 fatty acid and at least one xanthophyll selected from lutein and zeaxanthin, for use in the prevention or the treatment of the decline of a cognitive function of a subject or for use in the amelioration of a cognitive function of a subject, wherein the lutein is dosed to provide an amount of between about 0,2 mg and 15 mg of lutein per day and/or the zeaxanthin is dosed to provide an amount of between about 0,1 mg and 0,6 mg of zeaxanthin per day.

In particular, the at least one xanthophyll are the two xanthophylls lutein and zeaxanthin, according to the invention.

Typically, for the non-therapeutic method of the invention and/or for the composition for use in the prevention or the treatment of the decline of a cognitive function of a subject or for use in the amelioration of a cognitive function of a subject according to the invention, the at least one omega-3 fatty acid is dosed to provide an amount of between about 50 mg and 3 g of omega-3 fatty acid per day, preferably at least about 60 mg, more preferably at least about 75 mg, most preferably between about 120 mg and 600 mg.

It is preferred that for the non-therapeutic method of the invention and/or for the composition for use in the prevention or the treatment of the decline of a cognitive function of a subject or for use in the amelioration of a cognitive function of a subject according to the invention, the lutein is dosed to provide an amount of between 1,1 mg and 1,8 mg lutein per day and the zeaxanthin is dosed to provide an amount of between 0,17 mg and 0,30 mg zeaxanthin per day.

In an embodiment, in the non-therapeutic method according to the invention, the subject to whom the composition is administered is a healthy subject.

In an embodiment, in the non-therapeutic method according to the invention, the subject to whom the composition is administered is a human subject.

Preferably, in the non-therapeutic method according to the invention, the subject to whom the composition is administered is a healthy human subject.

It is part of the invention that the subject to whom the composition of the invention is administered, such as a healthy subject, is a woman or is a man. Typically, the composition of the invention is suitably administered to both men and women, according to the invention. Typically, the human subjects to whom the composition of the invention is administered are women and men such as healthy men and women, are typically elder subjects, about 60 years of age or older, such as at least about 65 years of age, or between about 65 and 80 years of age, such as about 70 years of age, or about 75 years of age. It is preferred that the human subjects to whom the composition of the invention is administered are subjects of up to 70 years of age, thus younger than 70 years of age. For example, the human subjects are between about 58 and 70 years of age such as about 64 years of age, or between 60 and 68 years of age.

In an embodiment, the composition for use in the prevention or the treatment of the decline of a cognitive function or for use in the amelioration of a cognitive function according to the invention, is administered to a subject, preferably a human subject. In a preferred embodiment, the composition for use in the prevention or the treatment of the decline of a cognitive function or for use in the amelioration of a cognitive function according to the invention is administered to a healthy subject, preferably a healthy human subject.

In one embodiment, the composition applied in the non-therapeutic method according to the invention or the composition for use according to the invention, is administered to a subject, whom has not been previously diagnosed with a memory disorder or with a cognitive impairment such as mild cognitive impairment, at the start of the administration of the composition to said subject.

In one embodiment, the composition applied in the non-therapeutic method according to the invention or the composition for use according to the invention, is administered to a subject, whom has not been diagnosed with a cognitive disorder or a neurological disorder such as a neurodegenerative disease selected from the group consisting of dementia and Alzheimer's disease.

In one embodiment, the administration of the composition to a subject according to the non-therapeutic method of the invention results in improving a cognitive function in said subject, wherein the cognitive function is any one or more of the group consisting of general alertness, motor speed, short-term memory such as short-term visual recognition memory, working memory, visual memory, new learning, sustained attention, and planning efficiency.

In a preferred embodiment, the administration of the composition to a subject according to the non-therapeutic method of the invention results in improving a cognitive function in said subject, wherein the cognitive function is any one or more of the group consisting of learning and memory, complex attention and executive functioning, preferably the improved cognitive function is at least two of said group of cognitive functions, more preferably the learning and memory, complex attention and executive functioning are all improved, when subjecting a subject to the method of the invention or when the composition is used for the prevention or the treatment of the decline of a cognitive function or used for the amelioration of a cognitive function according to the invention.

In one embodiment, administering the composition for use in the prevention or the treatment of the decline of a cognitive function or for use in the amelioration of a cognitive function according to the invention to a subject results in improving a cognitive function in said a subject, wherein the cognitive function is any one or more of the group consisting of general alertness, motor speed, short-term memory such as short-term visual recognition memory, working memory, visual memory, new learning, sustained attention, and planning efficiency.

In a preferred embodiment, administering the composition for use in the prevention or the treatment of the decline of a cognitive function or for use in the amelioration of a cognitive function according to the invention to a subject results in improving a cognitive function in said a subject, wherein the cognitive function is any one or more of the group consisting of learning and memory, complex attention and executive functioning, preferably the improved cognitive function is at least two of said group, more preferably the learning and memory, complex attention and executive functioning are all improved, when subjecting a subject to the method of the invention or when the composition is used for the prevention or the treatment of the decline of a cognitive function or used for the amelioration of a cognitive function according to the invention.

Thus in a preferred embodiment, by application of the non-therapeutic method according to the invention or the composition for use according to the invention administered to a subject, *e.g*. a healthy subject, results in improved cognitive function, wherein the cognitive function is any one or more of general alertness, motor speed, short-term memory such as short-term visual recognition memory, working memory, visual memory, new learning, sustained attention, and planning efficiency, preferably the cognitive function is any one or more of the group consisting of learning and memory, complex attention and executive functioning, more preferably the improved cognitive function is at least two of the cognitive functions of said group, most preferably the learning and memory, complex attention and executive functioning are all improved.

It is part of the invention that the improvements of cognitive function are assessed by conducting the well-established set of tests of Cambridge Neuropsychological Test Automated Battery (CANTAB). Preferably, human subjects are subjected to the non-therapeutic method of the invention, preferably, the human subjects are healthy human subjects. Preferably, the composition for use according to the invention is administered to human subjects, preferably healthy subjects, most preferably healthy human subjects.

Thus, the non-therapeutic method of the invention and the use of the composition in the prevention or the treatment of the decline of a cognitive function or for use in the amelioration of a cognitive function according to the invention, provide for improving cognitive function across many if not all cognitive domains related to cognition, *i.e.* learning and memory, social functioning, language, visuospatial function, complex attention, and executive functioning.

The CANTAB series of tests [reference: Robbins, T.W., James, M., Owen, A.M. et al. (1994) Cambridge Neuropsychological Test Automated Battery (CANTAB): a factor analytic study of a large sample of normal elderly volunteers. Dementia 5, 266-281] is applied according to the invention to assess the following cognitive functions with the test parameters as indicated:
- learning and memory: applied CANTAB tests are Spatial Span (SSP) forward and SSP reverse, Paired Associates Learning (PAL), Delayed Match to Sample (DMS)
- complex attention: applied CANTAB tests are Rapid Visual Information Processing (RVP), Choice Reaction Time (CRT)
- executive functioning: applied CANTAB test is Stockings of Cambridge (SOC)

Reference is made to the Examples section, Example 3, for a detailed summary of said CANTAB tests.

In one embodiment, learning and memory, complex attention and executive functioning improve for subjects, preferably human subjects, most preferably healthy human subjects, such as elder healthy human subjects over 60 years of age, after administering the composition of the invention according to the non-therapeutic method of the invention or after administering the composition for use in the treatment of cognitive function according to the invention, when said composition is administered at a daily basis for a time period of at least twelve months, preferably at least six months. Preferably, said composition comprises DHA, lutein and zeaxanthin. In particular, improved learning and memory, complex attention and executive functioning in subjects, preferably human subjects, most preferably healthy human subjects, such as elder healthy human subjects over 60 years of age, subjected to the non-therapeutic method of the invention or to whom the composition for use in the prevention or the treatment of the decline of a cognitive function of a subject or for use in the amelioration of a cognitive function of a subject according to the invention is administered, is established for at least six months, in particular for at least twelve months, such as for 6 to 12 months, according to the invention.

Thus, according to the invention, subjecting a subject to the non-therapeutic method of improving a cognitive function in said subject, results in improved learning and memory, complex attention and executive functioning in said subject, said improvements lasting for at least six to twelve months such as for six months or longer or such as for 12 months or longer. Thus, according to the invention, administering the composition of the invention for use in the prevention or the treatment of the decline of a cognitive function of a subject or for use in the amelioration of a cognitive function of a subject, to a subject results in improved learning and memory, complex attention and executive functioning in said subject, said improvements lasting for at least six to twelve months such as for six months or longer or such as for 12 months or longer. Exemplifying embodiments are provided in the Examples section here below, embodied for example in Table 4 depicting the results of a trial with human subjects to whom the composition of the invention was administered for a period of twelve months, resulting in improved cognitive function for the subjects.

According to the invention, it is preferred that human subjects, such as healthy human subjects, such as elder human subjects for example of over 60 years of age or for example up to 70 years of age or between 58 and 70 years of age, who are subjected to the non-therapeutic method of improving a cognitive function in said subject of the invention, are administered the composition of the invention comprising DHA, lutein and zeaxanthin, wherein the DHA is dosed to provide an amount of between about 120 mg and about 600 mg DHA per day, preferably about 170 mg DHA per day, the lutein is dosed to provide an amount of between about 1,1 mg and about 1,8 mg lutein per day, preferably about 1,1 mg lutein per day, and the zeaxanthin is dosed to provide an amount of between about 0,17 mg and about 0,30 mg zeaxanthin per day, preferably about 0,2 mg zeaxanthin per day.

According to the invention, it is preferred that human subjects, such as healthy human subjects, such as elder human subjects for example of over 60 years of age or for example up to 70 years of age or between 58 and 70 years of age, to whom the composition for use in the prevention or the treatment of the decline of a cognitive function of a subject or for use in the amelioration of a cognitive function of a subject of the invention is administered, receive the composition wherein said composition comprises DHA, lutein and zeaxanthin, wherein the DHA is dosed to provide an amount of between about 120 mg and about 600 mg DHA per day, preferably about 170 mg DHA per day, the lutein is dosed to provide an amount of between about 1,1 mg and about 1,8 mg lutein per day, preferably about 1,1 mg lutein per day, and the zeaxanthin is dosed to provide an amount of between about 0,17 mg and about 0,30 mg zeaxanthin per day, preferably about 0,2 mg zeaxanthin per day.

In particular, for the non-therapeutic method according to the invention comprising the administering of the composition, or for the composition for use according to the invention, the composition comprises lutein and zeaxanthin, wherein the lutein is dosed to provide an amount of about 1,1 mg lutein per day and the zeaxanthin is dosed to provide an amount of about 0,20 mg zeaxanthin per day. In particular, for the non-therapeutic method according to the invention comprising the administering of the composition, or for the composition for use according to the invention, the composition comprises at least one omega-3 fatty acid, which is docosahexaenoic acid and which is dosed to provide an amount of about 170 mg DHA per day.

In one embodiment, it is part of the non-therapeutic method according to the invention, that the subject to whom the composition is administered, has not been diagnosed with any one or more of a disorder affecting liver function or affecting kidney function, diabetes mellitus and hypertension.

In one embodiment, the composition for use in the prevention or the treatment of the decline of a cognitive function or for use in the amelioration of a cognitive function according to the invention, is for use with a subject who has not been diagnosed with any one or more of a disorder affecting liver function or affecting kidney function, diabetes mellitus and hypertension.

In one embodiment, in the non-therapeutic method according to the invention, the subject to whom the composition is administered, is diagnosed with macular degeneration selected from the group consisting of intermediate age-related macular degeneration and early age-related macular degeneration.

In one embodiment, the composition for use in the prevention or the treatment of the decline of a cognitive function or for use in the amelioration of a cognitive function according to the invention, is for use with a subject who is diagnosed with macular degeneration selected from the group consisting of intermediate age-related macular degeneration and early age-related macular degeneration.

In one embodiment, in the non-therapeutic method according to the invention, the subject to whom the composition is administered, is diagnosed with intermediate age-related macular degeneration or late age-related macular degeneration, corresponding to AMD graded as 3 or 4 on the AREDS scale. Particular improvements in the following four tests are seen when a subject diagnosed with intermediate age-related macular degeneration or late age-related macular degeneration is administered the composition of the invention: DMS, SSPforward, 'PAL total errors' test and 'SOC min problems solved' test.

In one embodiment, the composition for use in the prevention or the treatment of the decline of a cognitive function or for use in the amelioration of a cognitive function according to the invention, is for use with a subject who is diagnosed with intermediate age-related macular degeneration or late age-related macular degeneration, corresponding to AMD graded as 3 or 4 on the AREDS scale. Particular improvements in the following four tests are seen when the composition of the invention is administered to a subject diagnosed with intermediate age-related macular degeneration or late age-related macular degeneration: DMS, SSPforward, 'PAL total errors' test and 'SOC min problems solved' test.

In one embodiment, in the non-therapeutic method according to the invention, the subject is over 40 years old, preferably over 50 years old, more preferably over 60 years old, most preferably over 70 years old. Although the non-therapeutic method according to the invention is applicable for *e.g*. human subjects of any age, most pronounced beneficial effects with regard to improvement of cognitive function are arrived at when the method is applied with elderly subjects, such as subjects over 40 years old. Preferred are subjects who are otherwise healthy, who are over 55 years old, more preferably over 65 years old.

In one embodiment, the composition for use according to the invention is administered to a subject, wherein the subject is over 40 years old, preferably over 50 years old, more preferably over 60 years old, most preferably over 70 years old. It is preferred that the human subjects to whom the composition of the invention is administered are subjects of up to 70 years of age, thus younger than 70 years of age. For example, the human subjects are between about 58 and 70 years of age such as about 64 years of age, or between 60 and 68 years of age. Particular improvements in the following four tests are seen when a subject younger than 70 years of age such as about 64 years of age is administered the composition of the invention: the DMS test, the SSP forward test, the RVP B test and the 'SOC min problems solved test'. Particular improvements in the following four tests are seen when the composition of the invention is administered to a subject younger than 70 years of age such as about 64 years of age: the DMS test, the SSP forward test, the RVP B test and the SOC min problems solved test.

In one embodiment, the composition according to the invention is an aqueous composition.

In one embodiment, in the composition applied in the non-therapeutic method according to the invention or in the composition for use according to the invention, the at least one xanthophyll is contained in an aqueous dispersion.

In one embodiment, for the composition applied in the non-therapeutic method according to the invention or for the composition for use according to the invention, the aqueous dispersion comprised by the composition is selected from the group consisting of skimmed milk, semi-skimmed milk, buttermilk, a buttermilk fraction, fermented milk, yoghurt, soy drink, soy milk, fermented soy milk, fruit juices, fruit purees, syrups, vegetable juices, vegetable purees.

In one embodiment, for the composition applied in the non-therapeutic method according to the invention or for the composition for use according to the invention, the aqueous dispersion comprised by the composition is buttermilk or a buttermilk fraction.

The composition for use in the prevention or the treatment of the decline of a cognitive function or for use in the amelioration of a cognitive function according to the invention may be provided as a ready-for-use solution, as a stock solution from which the daily dose may be obtained, or may be prepared by dilution or it may be a dry composition, such as for example a powder or a crystalline composition, preferably a powder, from which a daily dose may be obtained by adding a fluid. The composition may also be used as dry matter and mixed with a foodstuff. The skilled person is well aware of these and other ways of administering a composition to a subject in need of the composition.

The xanthophyll in a composition for application in the non-therapeutic method of the invention or in a composition for use according to the invention may be contained in an aqueous or non-aqueous solution or in a dry form. It is however preferred that the xanthophyll is contained in an aqueous dispersion. Such a dispersion is contained in the term "aqueous composition" and "composition" according to the invention, and such a dispersion may be selected from the group consisting of skimmed milk, semi-skimmed milk, buttermilk, a buttermilk fraction, fermented milk, yoghurt, soy drink, soy milk, fermented soy milk, fruit juices, fruit purees, syrups, vegetable juices and vegetable purees. Preferably, the aqueous dispersion is buttermilk or a buttermilk fraction.

In a preferred embodiment, a composition for application in the non-therapeutic method of the invention or in a composition for use according to the invention comprises at least 20 mg of dairy polar lipids per kg of composition, such as at least 40, 60, 80, or 100 mg/kg. In a preferred embodiment, the composition comprises at least 200 mg of dairy polar lipids, such as 300, 400, 500, 600, 700, 800 or 900 mg per kg of composition. Whereas there is hardly any upper limit for the dairy polar lipid content of the composition, for practical purposes the dairy polar lipid content may be kept below 10.000 mg per kg of the composition.

It should be noted that the term 'buttermilk' as used herein refers to a dairy product obtained in a fermentation of a dairy product, such as whole milk. The use of what may be called 'synthetic buttermilk', such as acidified low fat milk or fat free milk is less preferred, although it is often labelled as buttermilk by dairy manufacturers. The fermentation process enriches buttermilk with the desirable polar lipids, which are present at only low levels or entirely absent in the synthetic buttermilk products. Generally, dairy products such as buttermilk, containing at least 80 mg of polar lipids per litre are suitable for use in the manufacture of the composition of the present invention. Preferred is the use of dairy products with an even higher dairy polar lipid content, such as at least 100 mg per litre, such as 120, 140, 180, 220, 260, 300, 340, 380 or even at least 420 mg per litre.

In a preferred embodiment, the xantophyll is contained in egg yolk. When chickens, *i.e.* laying hen, are fed with a diet enriched with xanthophylls, the yolk of their eggs contains increased amounts of these natural substances, which are found in the micelles. However, the amounts of xanthophylls that can be safely administered are on the one hand limited by the amount of xanthophylls contained in the egg yolk and on the other hand by the maximum amount of egg yolk that can be safely administered to a subject. In order to maximize the amount of xanthophylls that can be effectively delivered in the blood stream, strategies have been deployed to maximize the absorption of xanthophylls in the gut.

It has previously been described that the absorption in the gut is greatly enhanced by mixing the xanthophylls-containing egg yolk with polar lipids. For example certain dairy products are good sources of suitable polar lipids or phospholipids.

The egg yolk may be formulated as an aqueous dispersion, such as a dairy dispersion. The absorption in the gut of xanthophylls such as lutein and zeaxanthin is greatly enhanced by this formulation. Without wanting to be bound by theory, it is thought that this improved absorption is due to the formation of micelles that are present in the mixtures.

In an embodiment, in the non-therapeutic method according to the invention, a composition is administered to a subject, wherein the composition is dehydrated.

It is appreciated by the person skilled in the art that dehydration of a composition of the invention, such as an aqueous composition, can be achieved by applying one of many different technologies for dehydrating a composition. For example, in a preferred embodiment, the composition that is administered to a subject according to the method of the invention, is an aqueous solution or more preferred, an aqueous dispersion, which is dehydrated by applying spray drying such that a powder is provided.

Similarly, in an embodiment, the composition for use according to the invention is dehydrated, preferably by applying spray drying such that a powder is provided.

Thus, the compositions applied in the method of the invention or for use in the prevention or the treatment of the decline of a cognitive function or for use in the amelioration of a cognitive function according to the invention, as described herein, may also be in a concentrated, dehydrated or dry form, obtainable by dehydrating the aqueous compositions as described herein.

In one embodiment, in the non-therapeutic method according to the invention, the composition comprises egg yolk and an aqueous dispersion, wherein the egg yolk and the aqueous dispersion are present in the composition in a weight ratio of between 1:2 and 1:7, preferably between 1:2 and 1:3.

In one embodiment, the composition for use according to the invention comprises egg yolk and an aqueous dispersion, wherein the egg yolk and the aqueous dispersion are present in the composition in a weight ratio of between 1:2 and 1:7, preferably between 1:2 and 1:3, according to the invention.

The xanthophyll is preferably selected from the group consisting of zeaxanthin, lutein and meso-zeaxanthin and may be comprised in egg yolk. So the composition applied in the method of the invention or for use in the prevention or the treatment of the decline of a cognitive function or for use in the amelioration of a cognitive function according to the invention may comprise egg yolk or an egg yolk fraction containing the xanthophyll component of the egg yolk.

In one embodiment, the composition applied in the non-therapeutic method according to the invention or the composition for use according to the invention, is contained in a food product.

In one embodiment, the composition applied in the non-therapeutic method according to the invention or the composition for use according to the invention, is in the form of a dietary supplement.

In one embodiment, the composition applied in the non-therapeutic method according to the invention or the composition for use according to the invention, is in the form of a foodstuff, such as a drink, that has been enriched with the composition.

The composition according to the invention may advantageously be employed as a food product, as a food supplement, or as a medicament for the treatment of *e.g*. declining cognitive function. More in particular, it may be used in the prevention or the treatment of the decline of a cognitive function or for use in the amelioration of a cognitive function.

In one embodiment, in the non-therapeutic method according to the invention, the composition that is administered to a subject comprises an amount of lutein and/or of zeaxanthin such that the lutein is dosed to provide an amount of between about 0,2 mg and 15 mg of lutein per day, preferably between 0,25 mg and 10 mg per day, more preferably between 0,5 mg and 5,0 mg per day, most preferably between about 1,1 mg and 1,8 mg lutein per day, and/or the zeaxanthin is dosed to provide an amount of between about 0,1 mg and 0,6 mg of zeaxanthin per day, preferably between 0,12 mg and 0,5 mg per day, more preferably between 0,15 mg and 0,40 mg per day, most preferably between about 0,17 mg and 0,30 mg zeaxanthin per day. Typically, in the non-therapeutic method according to the invention, the composition that is administered to a subject comprises at least 0,2 mg zeaxanthin per daily dose. Typically, in the non-therapeutic method according to the invention, the composition that is administered to a subject comprises at least 1,1 mg lutein per daily dose.

In one embodiment, the composition for use in the prevention or the treatment of the decline of a cognitive function or for use in the amelioration of a cognitive function comprises an amount of lutein and/or of zeaxanthin such that the lutein is dosed to provide an amount of between about 0,2 mg and 15 mg of lutein per day, preferably between 0,25 mg and 10 mg per day, more preferably between 0,5 mg and 5,0 mg per day, most preferably between about 1,1 mg and 1,8 mg lutein per day, and/or the zeaxanthin is dosed to provide an amount of between about 0,1 mg and 0,6 mg of zeaxanthin per day, preferably between 0,12 mg and 0,5 mg per day, more preferably between 0,15 mg and 0,40 mg per day, most preferably between about 0,17 mg and 0,30 mg zeaxanthin per day. Typically, in the composition for use according to the invention, the composition that is administered to a subject comprises at least 0,2 mg zeaxanthin per daily dose, such as 0,2 mg zeaxanthin per daily dose. Typically, in the composition for use according to the invention, the composition that is administered to a subject comprises at least 1,1 mg lutein per daily dose, such as 1,1 mg lutein per daily dose. Thus, according to the invention, in the composition for use according to the invention, for example the composition that is administered to a subject comprises at least 0,2 mg zeaxanthin per daily dose, such as 0,2 mg zeaxanthin per daily dose and comprises at least 1,1 mg lutein per daily dose, such as 1,1 mg lutein per daily dose.

Preferentially, the lutein is dosed in the composition administered in the method of the invention or in the composition for use according to the invention, such that the lutein is dosed to provide an amount of between about 1 mg and 15 mg of lutein per day, preferably between 2 mg and 8 mg per day, more preferably between 3 mg and 6 mg per day, most preferably between about 3,3 mg and 5,0 mg lutein per day, according to the invention. Such doses of lutein are for example provided by applying in the composition egg yolk, such as egg yolk from poultry such as chicken, as a source of lutein, wherein the *e.g*. laying hens are fed with a diet enriched with lutein such that the lutein concentration in the egg yolk reached values applicable for the purpose of providing a composition comprising the afore mentioned lutein dose, according to the invention. The desired dosing of xantophylls, *e.g*. lutein and zeaxanthin, in the composition for use according to the invention and for administration to a subject in the method of the invention, is adjustable by selecting and implying in the composition the amount of egg yolk required to arrive at the desired dose.

Without wishing to be bound by theory, the bioavailability of lutein from composition NWT02 for application in the non-therapeutic method of the invention or for use according to the invention, is expected to be 5-7 times higher than lutein provided in capsules, related to the matrix of NWT02 in which lutein is provided.

A suitable daily dose of the composition for application in the non-therapeutic method of the invention or the composition for use according to the invention, for a human subject, *e.g*. a healthy human subject, is between about 5 gram (g) and 250 g, preferably between about 10 g and 200 g, such as between about 20 g and 100 g, such as 25 g, or 50 g or, preferably, 60 g. A skilled person is well aware of the recommended daily dose suitable for other subjects such as non-human animals.

In Table 1, preferred compositions applied in the method of the invention and preferred compositions applied for use in the prevention or the treatment of the decline of a cognitive function or for use in the amelioration of a cognitive function according to the invention, are provided.

**Table 1: Preferred compositions applied in the method of the invention or for use in the prevention or the treatment of the decline of a cognitive function or for use in the amelioration of a cognitive function according to the invention**

| **Ingredient:** | **Preferred ready for use [mg/kg composition]** | **Recommended daily dose [mg]** | **Preferred minim um conc. [mg/kg composition]** | **Preferred maximum conc. [mg/kg composition]** |
|---|---|---|---|---|
| Dairy polar lipids | 500 | 12 | 20 | 10.000 |
| Xanthophyll | 50 | 1 | 5 | 500 |
| Omega-3 fatty acid | 9.000 | 200 | 1.000 | 50.000 |

A xanthophyll content of at least 2 mg per kg of a composition for application in the non-therapeutic method of the invention or the composition for use according to the invention is preferred, preferably the composition comprises at least 3, 4, 5, 6, 8, 10, 15, 20, 30 or even at least 50 mg per kg. Preferred xanthophylls are lutein and zeaxanthin. In a further preferred embodiment, the composition comprises at least 10 mg lutein per kg composition, such as 12, 14, 16, 18 or at least 20 mg per kg, such as 25, 30, 35 or at least 40 mg per kg.

In a further preferred embodiment, the invention relates to a composition comprising at least 1.000 mg of omega-3 fatty acids such as DHA per kg composition, such as at least 2.000, 3.000, 4.000, 5.000, 6.000, 7.000, 8.000, 9.000 or even 10.000 mg per kg composition or more. Although there is no maximum for the DHA content of the composition, for practical purposes the composition may not contain more than 50.000 mg of DHA per kg of composition.

A preferred daily dose of the at least one omega-3 fatty acid, which is preferably DHA, is about 50 mg, such as 60 mg, 75 mg, 100 mg, such as 120 mg, 150 mg, 200, 400 or 500 mg, or 600 mg. In one embodiment, in the non-therapeutic method according to the invention, the composition that is administered to a subject comprises an amount of at least one omega-3 fatty acid, preferably DHA, such that the omega-3 fatty acid(s) is/are dosed to provide an amount of at least 170 mg per day, preferably more than 170 mg per day. Typically, the composition for use in the prevention or the treatment of the decline of a cognitive function of a subject or for use in the amelioration of a cognitive function of a subject according to the invention, comprises an amount of at least one omega-3 fatty acid, preferably DHA, such that the omega-3 fatty acid(s) is/are dosed to provide an amount of at least 170 mg per day, preferably more than 170 mg per day, such as about 170 mg per day.

It is preferred that the composition of the invention for application in the non-therapeutic method of improving a cognitive function in a subject of the invention, or the composition for use in the prevention or the treatment of the decline of a cognitive function of a subject or for use in the amelioration of a cognitive function of a subject according to the invention, comprises an amount of DHA, such that the DHA is dosed to provide an amount of at least 170 mg per day, such as about 170 mg per day, and comprises an amount of lutein such that the lutein is dosed to provide an amount of between 1,1 mg and 1,8 mg lutein per day, preferably at least 1,1 mg lutein per day such as 1,1 mg lutein per day and comprises an amount of zeaxanthin such that the zeaxanthin is dosed to provide an amount of between 0,17 mg and 0,30 mg zeaxanthin per day, preferably at least 0,17 mg zeaxanthin per day such as for example about 0,2 mg zeaxanthin per day.

Human subjects to whom the composition of the invention is administered in the non-therapeutic method of improving a cognitive function in said human subjects, such as healthy human subjects, such as elder subjects over 65 years of age or such as human subjects of between about 58 and 70 years of age such as about 64 years of age, and/or human subjects to whom the composition for use in the prevention or the treatment of the decline of a cognitive function of a subject or for use in the amelioration of a cognitive function of a subject of the invention is administered, such as healthy human subjects, such as elder subjects over 65 years of age or such as human subjects of between about 58 and 70 years of age such as about 64 years of age, benefit from improved visual matching ability and short-term visual recognition memory at least after six and twelve months of taking daily doses of the composition, according to the invention.

Compared to a matched control group of human subjects, human subjects taking a daily dose of the composition according to the invention benefit after six months and after twelve months at least from improved DMS (visual memory), improved SSP (working memory) and improved PAL (visual memory and new learning), according to the invention. Typically, improvements in DMS (visual memory), SSP (working memory) and PAL (visual memory and new learning) are about 27%, 33% and 43%, respectively, for human subjects taking the composition of the invention when subjected to the non-therapeutic method of the invention and/or for human subjects to whom the composition for use in the amelioration of a cognitive function of a subject of the invention is administered. Typically, such improvements are achieved for elder human subjects, *e.g*. between 55 and 100 years of age such as about 68 years of age or older, or about 75 years of age or older, or for example between about 58 and about 70 years of age such as between 60 and 68 years of age, 62 and 66 years of age or about 64 years of age. Typically, such improvements are achieved in healthy human subjects, *e.g*. both men and women.

Thus, by application of a composition of the invention comprising a daily dose of at least 50 mg DHA per day, such as between 50 mg and 3 g of omega-3 fatty acid per day, preferably at least about 60 mg, more preferably at least about 75 mg, most preferably between about 120 mg and 600 mg, such as preferably a daily dose of about 170 mg DHA per day, comprising lutein wherein the lutein is dosed to provide an amount of between 0,5 mg and 5,0 mg per day, more preferably between about 1,1 mg and 1,8 mg lutein per day, such as preferably a daily dose of about 1,1 mg lutein per day, and wherein the zeaxanthin is dosed to provide an amount of between 0,15 mg and 0,40 mg per day, more preferably between about 0,17 mg and 0,30 mg zeaxanthin per day, such as preferably a daily dose of about 0,2 mg zeaxanthin per day, human subjects benefit from improved DMS (visual memory), improved SSP (working memory) and improved PAL (visual memory and new learning), for a time period of at least six months, preferably at least 12 months, according to the invention, whereas the daily dose of lutein and/or zeaxanthin in the composition of the invention is lower than such doses applied in the art for compositions administered to human subjects for the purpose of influencing cognition, and whereas in the art effects of administering compositions comprising lutein and/or zeaxanthin and/or DHA to human subjects were only apparent for a shorter time period than now achieved, according to the invention. It is now part of the invention that administering the composition of the invention, said composition administered such that the daily dose with regard to lutein and/or zeaxanthin is relatively low, to human subjects result in a relatively prolonged beneficial effect on at least one or more of DMS, SSP and PAL, wherein said beneficial effect on at least one or more of visual memory, working memory, and visual memory and new learning, is apparent to a larger extent than is currently seen with compositions comprising lutein and/or zeaxanthin known in the art.

In the art, effects on cognition relating to administering a composition comprising lutein and/or zeaxanthin at higher dose than present in the composition of the invention, have been reported relating to a time period of 90 days or 4 months, for example.

Typically, in the art, daily intake of lutein and/or zeaxanthin was 12 mg per day lutein, and about 0,5 mg zeaxanthin per day. Typically, in the art, after 4 months, the improvement in the shopping list memory test was about 19% and the improvement in the MIR Apartment memory test was about 9% when human subjects were administered such a composition known in the art, comprising 12 mg lutein, about 0,5 mg zeaxanthin in a daily dose and further comprising 800 mg DHA per daily dose.

The shopping list memory test, or Shopping list task, aims at testing short-longer term memory by verbal recall through reading ten associated words (common food items found in a supermarket) to the subjects in up to five verbally presented serial trials. The Memory In Reality (MIR) test, or MIR Apartment memory test, aims at testing short-longer term memory by verbal and visuospatial (location) recall after a delay by placing ten common household objects in seven rooms of a model apartment. Such tests are developed to determine effects of treatments, effects of administering a composition to a subject, *etc.,* on parameters relating to cognition, similar or the same to the purpose of the tests applied for the human subjects to whom the composition of the invention was administered as part of the non-medical method of the invention, or for use in the prevention or the treatment of the decline of a cognitive function of a subject or for use in the amelioration of a cognitive function of a subject, the subject being a man or a woman, such as elder men and women, such as healthy elder subjects over 60 years of age such as for example between 60 and 70 years of age such as about 64 years of age, according to the invention (see exemplifying embodiments, in the Examples section, hereunder).

The invention is further illustrated by the following examples.

### EXAMPLES

### Example 1: production of a composition comprising a xanthophyll.

A composition comprising a xanthophyll and an omega-3 fatty acid was produced from eggs obtained by feeding lutein and zeaxanthin to chickens and collecting the yolks from eggs produced by these chickens (reference is made to the method of production and Example 1 in WO 2009/078716). These eggs are referred to as enriched eggs herein.

Feeds for producing the enriched eggs were formulated and produced within the legal requirements for animal feed. The use of lutein and zeaxanthin is regulated under EU regulation 1831/2003. The dosage of lutein and zeaxanthin in feed did not exceed the legal limit of 80 ppm in animal feed.

Enriched eggs produced by poultry that were fed the lutein and zeaxanthin enriched feed contained 45 - 80 mg lutein per kg egg yolk and 10-30 mg zeaxanthin per kg egg yolk.

The animals were also fed a diet rich in omega-3 fatty acids; the eggs contained approximately 200 mg omega-3 fatty acids per kg egg yolk, with a standard deviation of 10 mg.

### Example 2: Manufacturing of an aqueous dispersion comprising xanthophylls and omega-3 fatty acid.

Eggs enriched with xanthophylls and the omega-3 fatty acid DHA were produced under an ISO 22000/HACCP certified quality scheme as described in Example 1. Eggs were separated in yolk and albumen in an automated facility in an ISO 22000:2005 certified plant. Egg yolk from 165,000 enriched eggs was mixed (15 min., 4°C) with 5,500 liters of buttermilk containing 80 mg polar lipids per liter, and 170 kg of sugar. The liquid was pasteurized for 3 minutes at 65°C and cooled to 4°C. This composition is herein referred to as NWT02 or NWT02 liquid formulation.

For storage, the composition was dried to a powder with 4(±1)% moisture content and mixed with free flowing agent (SiO₂, Sipernat 22S).

### Example 3 : analysis of the influence of a composition comprising at least one omega-3 fatty acid and at least one xanthophyll on cognitive function in human subjects.

The primary objective of a clinical trial hereunder outlined was to assess cognitive function in human subjects who were administered a composition comprising at least one omega-3 fatty acid and at least one xanthophyll, by psychometric testing. The at least one omega-3 fatty acid was DHA, the at least one xanthophyll were lutein and zeaxanthin. The clinical trial is executed at Tufts University (Boston, MA, USA).

### Study design

The study is a single-centre, double-blind, randomized, placebo-controlled trial designed to test the effect of a dairy beverage containing eggs which have been enriched for lutein, zeaxanthin and DHA, on cognitive function in human subjects. The subjects had early or intermediate signs of AMD. The beverage was supplied as a dry powder and was prepared by the addition of water.

Subjects: Women and men (N = 13, age (standard deviation) 74.0 (15.1) years in Group A (n = 6 subjects; 5 female, 1 male) and 68.8 (7.4) years in Group B (n = 7 subjects; 4 female, 3 male), BMI 18-35 kg/m²) were recruited at the site. Two weeks before the study subjects underwent a screening examination that includes a medical history, a physical examination and a fasting blood sample was drawn from the subject as a check for clinical chemistries. In addition, serum cholesterol, lipoproteins and triglycerides could be determined at this screening visit or at Visit 1. Subjects were given a food frequency questionnaire for determination of dietary intakes. Volunteers with any history or biochemical evidence of liver, kidney, or pancreatic disease, anemia, active bowel disease or resection, atrophic gastritis, easy bruising or bleeding, bleeding disorders, hyperglyceridemia, hyperlipidemia, diseases that interfere with fat absorption, or alcoholism were excluded from the study.
Moreover, individuals taking medication suspected of interfering with fat absorption, like bile sequestrants, were excluded. Subjects were excluded if they have had regular use of carotenoid, fish oil or omega-3 fatty acid supplement within 1 month before admission into the study.

Before the beverage supplementation was initiated, subjects were given an ophthalmic exam. Subjects had AMD, and had at least one eye (designated the "study eye") being aligned with AREDS category 2 or 3 definitions. Subjects with a history of other eye diseases (glaucoma, cataract) were excluded. Smoking was not be permitted during the course of the study. Subjects in the clinical trial provided informed consent.

At t = 0, t = 6 months and t = 12 months, a Food Frequency Questionnaire (FFQ) was filled in by the human subjects in the trial. Furthermore, at the same time points, Cognitive measures were assessed (see below).

### STUDY POPULATION

### Population (base)

13 healthy subjects (women and men) with early signs of AMD, aged 50 years and older.

### Inclusion criteria

- Early AMD (AREDS category 2)
   o many small drusen, or
   o a few intermediate-sized (63-124 micrometres in diameter) drusen, or macular pigmentary changes,
   or
- Intermediate AMD (AREDS category 3)
   ∘ extensive intermediate sized (63-124 micrometres in diameter) drusen, or
   ∘ at least one large (>125 micrometres in diameter) drusen or
   ∘ geographic atrophy not involving the foveal centre
- men and women age >50 years
- BMI 18-35 kg/m2
- Vision > 20/40 for Snellen visual acuity
- lutein intake of < 2 mg/day (including supplements)
- DHA intake of < 150 mg/day (including supplements)
- must be able to give written informed consent
- have normal hematologic parameters
- normal values of plasma albumin
- normal values for liver and kidney function
- no use of carotenoid, fish oil, or n3 fatty acid supplements (within 1 month of study start)

### Exclusion criteria

- ocular media opacity (severe cataract)
- history of active small bowel disease or resection
- atrophic gastritis
- history of hyperlipidemia or screening values as follows (LDL > 5.33mmol/L or 205mg/dL; triglycerides > 4.52mmol/L or 400 mg/dL)
- hypertension (>150/90 mm Hg)
- diabetes mellitus
- alcohol intake of >2 drinks/day or 14 drinks/week
- pancreatic disease
- dementia or Alzheimer's disease
- anemia, and bleeding disorders
- known allergy to egg or egg products
- known allergy to milk or milk products
- known allergy to cocoa or chocolate products
- known allergy to fish or fish oils
- lactose intolerance
- pregnancy or lactation
- diseases that interfere with fat absorption, e.g. colitis, celiac disease, Crohn's disease, cystic fibrosis (as determined by screening interview)
- medication or supplements that contain a significant level of carotenoids, including an amount of lutein of more than 0.25 mg per day
- medications that interfere with fat absorption, e.g. bile sequestrants (as determined by screening interview)
- use of antipsychotic, antimanic, or dementia medications
- smoking or use of nicotine patches or gum (within the past 6 months)
- subjects having extremely high dietary intakes of carotenoids
- stroke, head injury with loss of consciousness or seizures
- For US and UK center: Non English speaking

### TREATMENT OF SUBJECTS

### Investigational product/treatment

The intervention is based on egg yolk containing lutein, zeaxanthin and DHA, combined with buttermilk. The egg yolk is collected following the breaking of eggs, which have been enriched for lutein, zeaxanthin and DHA (See Example 1, Example 2). The dairy beverage is pasteurized and subsequently dried at large scale by spray-drying. To the resulting powder an aroma is added, which results in a chocolate taste. The powder is packaged in sachets of approximately 25 gram. The 25-gram package comprises the daily dose of lutein, zeaxanthin and DHA, which is at least 1,1 mg, at least 0,2 mg and at least 170 mg, respectively.

Each morning, the subjects were requested to dissolve the contents of one sachet in 60 ml water. Subjects were provided with small bottles. The subjects needed to put the water in the bottle, add the powder and shake the bottle prior to drinking the contents; the beverage had to be consumed shortly after preparation.

The matching control (placebo) beverage was also be made out of powder supplied in sachets and had a similar nutritional load (calories) and organoleptic properties.

### INVESTIGATIONAL PRODUCT

Lutein, zeaxanthin and DHA are well established food ingredients and do not represent medicinal products. Furthermore, these ingredients are widely used in food and food supplements.

Lutein and zeaxanthin are naturally occurring substances which are present in many food products, especially in green leafy vegetables such as spinach.

Docosahexaenoic acid (DHA) is an omega-3 fatty acid that is a primary structural component of the human brain cerebral cortex, sperm, testicles and retina. It can be obtained directly from fish oil. Fish is a common natural source of DHA in regular food.

### Description and justification of route of administration and dosage

The intervention product contains egg yolk of a lutein/zeaxanthin/DHA-enriched egg. Research has shown that egg yolk is an effective and efficient vehicle for the absorption of lutein and zeaxanthin. Both the intervention and control product are based on a dry powder.

By dissolving the powder from the sachets in water, a drink of 60 ml is prepared which was easily swallowed and digested.

Previous studies have shown the beverage formulations to be suitable for daily consumption, have a high bioavailability and achieve adequate plasma concentrations of both lutein and zeaxanthin with a limited increase in plasma cholesterol levels.

Clinical program to date has shown that all beverages containing the enriched egg-yolk - both dried versions and the liquid one - were able to significantly increase serum lutein levels after 6 weeks intervention compared to the control beverage. The beverage containing the enriched egg-yolk increased serum lutein levels to a greater extent compared to the beverage prepared starting from dried individual components. No significant difference was seen in the effect on serum lutein levels between the lutein-enriched egg-yolk beverage NWT02 and the beverage obtained starting from dried components.

### Dosages, dosage modifications and method of administration

Test subjects consumed the test products with a volume of 60 ml every morning for a period of one year. The intervention product (based on buttermilk) contained egg yolk in the beverage.

The level of lutein, zeaxanthin and DHA in the final product has to be stated as an approximate dosage as the eggs are produced in a natural way and as such the concentration will vary to a limited extent between the eggs. However, the concentration of the carotenoids lutein and zeaxanthin in the eggs has been measured repeatedly and shows only limited variation in concentration.

**Informed consent and obtaining of consent:** Informed consent was obtained during the screening visit by staff member at the site. Subjects who passed the screening were invited to participate in the study. Informed consent to participate in the study was obtained from the subjects by the study nurse or relevant staff member.

### Laboratory tests:

Fasting blood was drawn into vacutainers. The samples for the safety analysis were processed according to the instructions of the local labs, which performed the safety analysis.

The samples for the analysis of the carotenoids needed to be spun down immediately and needed to be stored at -70 degrees Celsius until they were sent to the central lab for analysis.

### Safety panel (at screening and at month 12)

The safety panel included the following parameters and were assessed locally:
Hematology: Hemaglobin and Hematocrit
Biochemistry: Sodium, potassium, creatinin, bilirubin, albumin, alkalic phosphatase, gamma-glutamyltranspeptidase, ASAT and ALAT

### Carotenoids (at either -1 or 0 months, 6 months and 12 months)

The carotenoids included the following parameters and were assessed:
Lutein and zeaxanthin.

These samples were collected, protected from light, and centrifuged within 1 h (15 min, at 1.000xg, 4°C) to separate serum. Aliquots were stored (at -70°C) until analyses.

### Lipid profile (at either -1 or 0 months, 6 months and 12 months)

The lipid profile included the following parameters and were assessed:
Total cholesterol, LDL, HDL and triglycerides.

### Description study procedures

### Cognitive Function

Cognitive function was assessed using the standardized interactive test system "CANTAB" as developed and provided by Cambridge Cognition Ltd (Tunbridge Court, Tunbridge Lane, Bottisham, Cambridge, UK). It is a computerized cognitive assessment battery that has been extensively evaluated for reliability and validity, and has been demonstrated to be a sensitive and specific cognitive assessment tool. CANTAB has a track-record in the assessment of age-related cognitive decline and has been used in studies of dietary supplementation and cognition. Scores on a test of estimated premorbid intelligence and questionnaires of fatigue and mood were used as covariates in the data analysis and to assess comparability of groups.

**Subject timeline:** Subjects visited the Study center on study visits at screening and at start of the intervention (0 months), after 6 months and 12 months for the fasting blood sample and to perform the various assessments.
At the 0 and 6 month visit they also picked up supplies of test or placebo beverage. A 2 month supply of beverage could be collected by or separately supplied to the participants on study months 2, 4, 8, and 10. Participants were instructed on how to prepare the beverage from the dry powder by the research dietician or other staff members. Participants took their beverage daily for 12 months.

### Explanation of Cambridge Neuropsychological Test Automated Battery (CANTAB) and its subtests measured in the clinical trial

All tests performed are subtests of the Cambridge Neuropsychological Test Automated Battery (CANTAB). This test is computerised, which increases data accuracy and reliability over classical pencil-paper test and is a widely used, validated, and reliable neuropsychological battery [Robbins *et al.,* 1994]. The test results in a comprehensive, detailed and sensitive assessment of cognitive performance. Below, and in Table 2, a subset of tests comprised by CANTAB are detailed, which tests are applied in the clinical trial for subjects of Group A and Group B, at the start of the trial and at time-points 6 months and 12 months. Group A received placebo; Group B received the composition according to the non-therapeutic method of the invention and for use according to the invention, referred to as NWT02

The following **Attention Tests** were performed.
**1.** The **Choice Reaction Time (CRT)** task is a compound measure of processing speed and attentional capacity. The CRT task is used to study selective attention, or the ability to filter out irrelevant information. CRT is a 2-choice reaction time test that is similar to the simple reaction time test. However, here, stimulus and response uncertainty are introduced using two possible stimuli and two possible responses.
   An arrow-shape is displayed on either the left or the right side of the screen. The participant must select the left-hand button if the stimulus is displayed on the left-hand side of the screen, and the right-hand button if the stimulus is displayed on the right-hand side of the screen. The outcome measure is 'CRT mean latency', which is the mean latency (response speed) of the response from stimulus appearance to button press.
   Domain: general cognitive tests
**2. Rapid Visual Information Processing (RVP)** is a measure of sustained attention and processing speed.
   During this test, a white box appears in the centre of the computer screen with digits from 2-9 appearing in pseudo-random order at the rate of 100 digits per minute. Subjects were asked to detect target sequences of three digits (for example 2-4-6, 3-5-7) and to register responses using the press pad. The outcome measure was the signal detection of the strength of trace required to elicit a response.
   Domain: attention and psychomotor speed tests

### Visual Memory Tests

**1.** The **Delayed Match to Sample (DMS)** test assesses both simultaneous visual matching ability and short-term visual recognition memory, for non-verbalisable patterns.
   A complex visual pattern (the sample) is shown to the participant, which is both abstract and non-verbal, followed by four similar patterns, after a brief delay. The participant must select the pattern which exactly matches the sample. In the present research the number of correct patterns selected was the outcome measure.
   Domain: memory
**2. Paired Associates Learning (PAL)** assesses visuospatial associative learning and episodic recall memory. The test is very sensitive to medial temporal lobe dysfunction.
   During this test, boxes are presented on a screen and opened in randomised order with one or more containing a pattern. The patterns are then displayed on the middle of the screen, one at a time, and the subject must touch the box where the pattern was originally located. If the participant makes an error, the boxes are opened in sequence again to remind the participant of the locations of the patterns. The total number of errors and stages completed are used as outcome for this test.
   Domain: memory

### Executive Function, Working Memory and Planning Tests

**1. Spatial Span (SSP) forward and SSP reverse** assess visuospatial working memory capacity.
   White squares are shown on the screen, some of which briefly change color in a variable sequence. The participant must then select the boxes which changed color in the same order that they were displayed by the computer (for the forward variant) or in the reverse order (for backward variant). The number of boxes in the sequence increases from two at the start of the test, to nine at the end and the sequence and color are varied through the test.
   Domain: executive function
**2. Spatial Working Memory (SWM)** assesses an individual's ability to retain spatial information and to manipulate remembered items in working memory. Spatial working memory entails the ability to keep spatial information active in working memory over a short period of time. The test is a sensitive measure of frontal lobe and executive dysfunction and provides a measure of strategy as well as working memory errors.
   The test begins with a number of colored squares (boxes) shown on the screen. The aim of this test is that by selecting the boxes and using a process of elimination, the participant should find one yellow 'token' in each of a number of boxes and use them to fill up an empty column on the right-hand side of the screen. Depending on the difficulty level used for this test, the number of boxes can be gradually increased until a maximum of 12 boxes are shown for the participants to search. The color and position of the boxes used are changed from trial to trial to discourage the use of stereotyped search strategies.
   Spatial Working Memory requires retention and manipulation of visuospatial information.
   Domain: memory
**3. Stockings of Cambridge (SOC)** is a test to assess various of frontal-executive functions such as spatial planning, thinking and problem solving ability.
   The participant is shown two displays containing colored balls. The displays are presented in such a way that they can be easily perceived as stacks of colored balls held in stockings or socks suspended from a beam. The participant must move the balls in the lower display to copy the pattern shown in the upper display. The balls may be moved one at a time by selecting the required ball, then selecting the position to which it should be moved. The participant is instructed to make as few moves as possible to match the two patterns.
   Domain: general cognitive tests

**Table 2: Cognitive Test Battery - according to CANTAB**

| **Cognitive Test** | **Description** | **Outcome Measures** |
|---|---|---|
| Attention Tests | | |
| Choice Reaction Time (CRT) | 2-choice reaction time test with two possible stimuli and two possible responses | Mean correct latency (response speed) |
| Rapid Visual Information Processing (RVP) | Test of sustained attention | B" - Signal detection measure of the strength of trace required to elicit a response |

| Visual Memory Tests | | |
|---|---|---|
| Delayed Match to Sample (DMS) | Test of simultaneous and delayed matching to sample | Percent correct (all delays) |
| Paired Associates Learning (PAL) | Assesses visual memory and new learning | Total errors (adjusted) and Stages completed |

| Executive Function, Working Memory and Planning Tests | | |
|---|---|---|
| Spatial Span (SSP) | Assesses working memory | Span length |
| SSP Reverse | Assesses working memory | Span length |
| Spatial Working Memory (SWM) | Tests ability to retain spatial information and manipulate items in working memory | Between errors and Strategy |
| Stockings of Cambridge (SOC) | Test of spatial planning and spatial working memory | Problems solved in minimum moves Mean number of moves for 3, 4, and 5 move problems |

### REFERENCE

1. Robbins TW, James M, Owen AM et al. (1994) Cambridge Neuropsychological Test Automated Battery (CANTAB): a factor analytic study of a large sample of normal elderly volunteers. Dementia 5, 266-281.

### RESULTS OF THE CLINICAL TRIAL

Table 3 summarizes the characteristics of the human subjects, which were otherwise healthy human subjects, who entered the clinical trial for assessing the influence of the composition according to the invention on improvement of cognitive function.

**Table 3. Baseline characteristics of subjects in Group A (n=6) and B (n=7) of 12 month study.**

| | **Group A** | **Group B** | **P value** |
|---|---|---|---|
| Age, years, mean (SD) | 74.0 (15.1) | 68.6 (7.4) | 0.42 |
| Education (1-5)¹ | 2.50(1.8) | 3.14 (1.35) | 0.47 |
| Sex (female/male) | 5/1 | 4/3 | 0.55 |

| | | | |
|---|---|---|---|
| ¹0: 0-8^{th} grade; 1: high school; 2: high school graduate; 3: some college (includes Associate's degree, technical degree, apprenticeship); 4: college graduate (4 year degree); 5: graduate degree. REMARK: Group A received placebo; Group B received the composition according to the non-therapeutic method of the invention, referred to as NWT02. | | | |

In Tables 4-6, the cognitive scores for Group A and Group B at t = 0 (start of daily intake of placebo or NWT02, respectively), t = 6 months and t = 12 months, for the indicated subset of CANTAB tests, are provided.

**Table 4. Cognitive scores in subjects at baseline (0 month) and after 6 and 12 months in groups A (n=6) and B (n=7). Mean (SD).**

| | Group A | | | Group B | | |
|---|---|---|---|---|---|---|
| | baseline | 6 mo | 12 mo | baseline | 6 mo | 12 mo |
| CRT *mean latency msec* | 404.2 (42.1) | 400.0 (67.8) | 385.1 (52.1) | 429.4 (95.2) | 423.8 (136.2) | 379.0 (63.7) |
| DMS *percent correct all delays¹* | 78.9 (10.7) | 72.2 (11.5) | 78.9 (12.9) | 71.4 (13.7) | 84.8 (12.6) | 77.1 (9.3) |
| SSP Forward *highest span* | 5.6 (1.5) | 5.2 (0.4) | 4.7 (0.5) | 4.9 (0.9) | 5.1 (0.9) | 5.7 (1.3) |
| SSP Reverse *highest span* | 5.0 (1.1) | 4.7 (1.0) | 4.7 (0.8) | 4.6 (1.4) | 4.7 (1.3) | 5.0 (1.5) |
| PAL *total errors (adjusted)* | 37.0 (17.4) | 37.3 (26.5) | 40.7 (21.9) | 24.9 (12.0) | 34.0 (26.9) | 16.7 (12.4) |
| PAL *stages completed²* | 2.8 (0.4) | 3.0 (0.9) | 2.8 (0.8) | 3.6 (0.5) | 3.1 (1.1) | 3.7 (0.5) |
| SWM *errors* | 56.7 (17.4) | 54.5 (19.6) | 48.2 (22.5) | 53.1 (18.7) | 55.7 (21.6) | 50.0 (23.6) |
| SWM *strategy* | 31.2 (4.0) | 28.8 (4.4) | 31.0 (3.4) | 28.4 (6.6) | 30.0 (6.7) | 29.9 (7.5) |
| RVP *signal detection* | 0.85 (0.15) | 0.78 (0.26) | 0.79 (0.16) | 0.81 (0.32) | 0.80 (0.33) | 0.78 (0.27) |
| SOC # *completed in minimum moves* | 7.3 (1.6) | 7.7 (3.1) | 7.5 (2.7) | 7.1 (2.5) | 8.1 (2.1) | 9.1 (2.3) |
| SOC *mean* # *of moves, 3 moves* | 3.6 (1.2) | 3.7 (0.6) | 3.6 (0.8) | 3.4 (0.6) | 3.3 (0.4) | 3.1 (0.2) |
| SOC *mean* # *of moves, 4 moves* | 5.3 (0.8) | 5.3 (1.4) | 5.2 (1.3) | 5.5 (1.2) | 5.5 (0.8) | 4.9 (1.3) |
| SOC *mean # of moves, 5 moves³* | 8.3 (1.6) | 7.3 (2.0) | 7.3 (1.7) | 7.6 (1.0) | 6.8 (1.3) | 6.5 (1.1) |

| | | | | | | |
|---|---|---|---|---|---|---|
| CRT: Choice Reaction Time; DMS: Delayed Matching to Sample; SS: Spatial Span; PAL: Paired Associates Learning; SWM: Spatial Working Memory (higher scores are worse); RVP: Rapid Visual Information Processing (higher scores are better); SOC: Stockings of Cambridge ¹Group B tended to be different from baseline at 6 months (P=0.08) ²Group A and Group B differed at baseline ³Group B significantly differed from baseline at 12 months (P=0.049) | | | | | | |

**Table 5. Change in cognitive scores from baseline at 6 and 12 months in subjects from groups A (n=6) and B (n=7). Mean (SD).**

| | Group A | | Group B | |
|---|---|---|---|---|
| | 6 mo | 1 2 mo | 6 mo | 12 mo |
| CRT *mean latency msec* | -4.2 (54.6) | -19.1 (43.4) | -5.6 (122.7) | -50.4 (80.4) |
| DMS *percent correct all delays¹* | -6.7 (15.8) | 0.0 (13.3) | 13.3 (14.4) | 5.7 (15.6) |
| SSP Forward *highest span²* | -0.4 (1.7) | -0.8 (1.3) | 0.3 (0.8) | 0.9 (0.7) |
| SSP Reverse *highest span* | -0.3 (0.5) | -0.3 (0.5) | 0.1 (1.1) | 0.4 (1.0) |
| PAL *total errors (adjusted)²* | 0.3 (13.4) | 3.7 (9.5) | 9.1 (17.7) | -8.1 (6.7) |
| PAL *stages completed* | 0.2 (0.8) | 0.0 (0.6) | -0.4 (0.5) | 0.1 (0.4) |
| SWM *errors* | -2.2 (8.9) | -8.5 (15.6) | 2.6 (13.9) | -3.1 (13.7) |
| SWM *strategy¹* | -2.3 (1.9) | -0.2 (1.8) | 1.6 (2.4) | 1.4 (1.5) |
| RVP *signal detection* | -0.07 (0.2) | -0.06 (0.1) | -0.01 (0.1) | -0.03 (0.2) |
| SOC # *completed in minimum moves* | 0.3 (2.1) | 0.2 (1.7) | 1.0(1.6) | 2.0 (2.2) |
| SOC *mean* # *of moves, 3 moves* | 0.1 (0.9) | 0.0 (0.7) | -0.1 (0.5) | -0.4 (0.6) |
| SOC *mean* # *of moves, 4 moves* | 0.0(1.5) | -0.1 (1.5) | 0.0 (1.2) | -0.6 (1.9) |
| SOC *mean* # *of moves, 5 moves* | -1.0 (2.4) | -0.9 (1.2) | -0.9 (1.1) | -1.2 (0.9) |

| | | | | |
|---|---|---|---|---|
| CRT: Choice Reaction Time; DMS: Delayed Matching to Sample; SS: Spatial Span; PAL: Paired Associates Learning; SWM: Spatial Working Memory (higher scores are worse); RVP: Rapid Visual Information Processing (higher scores are better); SOC: Stockings of Cambridge ¹Change from baseline significantly different between groups at 6 months (both P<0.05) ²Change from baseline signficantly different between groups at 12 months (both P=0.02) | | | | |

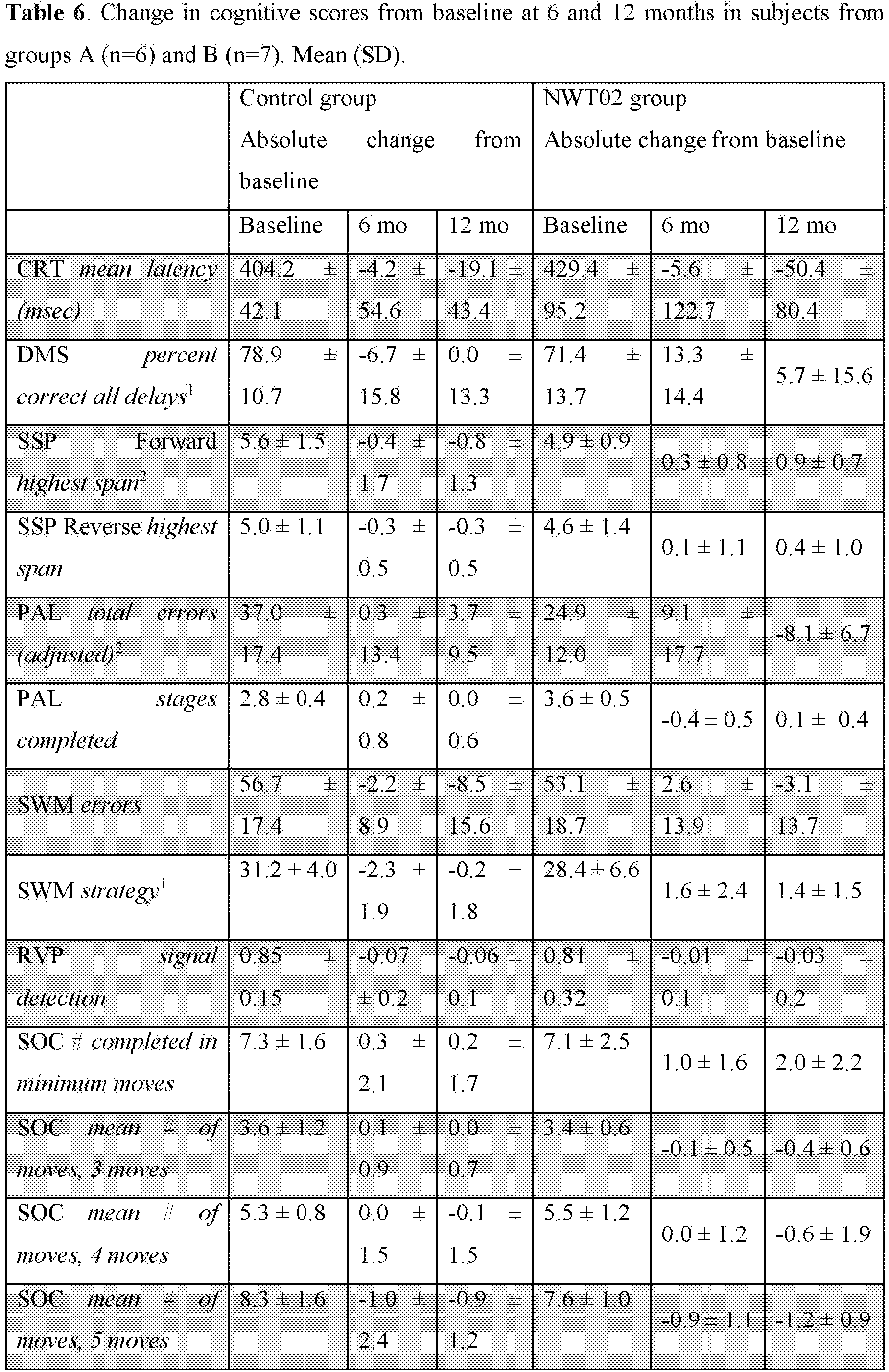

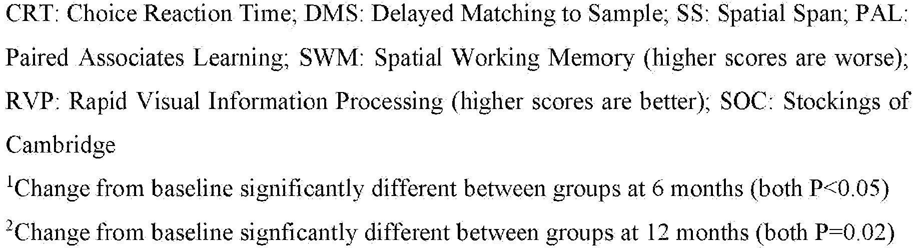

In brief, the clinical trial data are summarized as follows:
**DMS (visual memory, change, *i.e.* improvement, in NWT02 group versus placebo at 6 months: 27%):** The participant is shown a complex visual pattern, that is both abstract and non-verbal (the sample), followed by four similar patterns, after a brief delay. The participant must select the pattern which exactly matches the sample. In some trials the sample and the choice patterns are shown simultaneously, in others there is a delay (of 0, 4 or 12 seconds) before the four choices appear.
**SSP (visuospatial working memory, change, *i.e.* improvement, in NWT02 group versus placebo at 12 months: 33%):** White squares are shown on the screen, some of which briefly change color in a variable sequence. The participant must then select the boxes which changed color in the same order that they were displayed by the computer (for the forward variant) or in the reverse order (for backward variant). The number of boxes in the sequence increases from two at the start of the test, to nine at the end and the sequence and color are varied through the test.
**PAL (visual memory and new learning, change, *i.e.* improvement, in NWT02 group versus placebo at 12 months: 43%);** Boxes are displayed on the screen and are "opened" in a randomized order. One or more of them will contain a pattern. The patterns are then displayed in the middle of the screen, one at a time and the participant must select the box in which the pattern was originally located, If the participant makes an error, the boxes are opened in sequence again to remind the participant of the locations of the patterns. Increased difficulty levels can be used to test high-functioning, healthy individuals.

All parameters mentioned here above in the summary were in the 'memory' domain of cognition and reached a statistical significance of P < 0.05.

Based on the data set obtained for Group A and Group B over a period of 12 months, the following results were obtained with regard to the indicated CANTAB tests. Group A received placebo; Group B received the composition according to the non-therapeutic method of the invention, referred to as NWT02 (See Example 1, 2).
**general alertness:** choice reaction time (CRT), improvement versus placebo at t=6 months and t=12 months
**motor speed:** choice reaction time (CRT), improvement versus placebo at t=6 and t=12
**memory, such as short-term memory or visual recognition memory:** delayed match to sample (DMS), prevention of decline (improvement) at t=6 and t=12
**working memory:** Spatial Span (SSP) forward and backward AND Stockings of Cambridge (SOC), prevention of decline (improvement) at t=6 and t=12
**memory, such as visual memory and new learning:** Paired Associates Learning (PAL), improvement at t=12
**new learning:** Paired Associates Learning (PAL), improvement at t=12
**sustained attention:** Rapid Visual Information Processing (RVP), inhibition of delay at t=6 and t=12 for Group B compared to placebo
**planning efficiency,** also referred to as 'spatial working memory and spatial planning': Stockings of Cambridge (SOC), improvement at t=6 and t=12 for SOC completed in minimum moves, and mean number of moves 3 and 5 moves, improvement at t=12 for SOC mean number of moves, 4 moves.

The CANTAB tests thus show that the beneficial effect of the composition of the invention on cognitive function, *i.e*. improvement of cognitive function upon administering the composition for up to 12 months by human subjects, are as follows:
- learning and memory: applied CANTAB tests are Spatial Span (SSP) forward and SSP reverse, Paired Associates Learning (PAL), Delayed Match to Sample (DMS)
- complex attention: applied CANTAB tests are Rapid Visual Information Processing (RVP), Choice Reaction Time (CRT)
- executive functioning: applied CANTAB test is Stockings of Cambridge (SOC)

In Table 7, the beneficial effects of the test composition NWT02 are summarized. A clear and pronounced beneficial effect, *i.e.* an improvement of cognitive function, is obtained after six and twelve months of administering NWT02 to test Group B, compared to placebo Group A. By taking NWT02 on a daily basis for up to twelve months, the healthy subjects of Group B benefit from improved learning and memory, improved complex attention and improved executive functioning. Either said cognitive functions are improved on an absolute scale when compared to baseline values at the start of the clinical trial, or said cognitive functions are improved when compared to the placebo Group A, in that a cognitive function declined to a lesser extent in Group B over time.

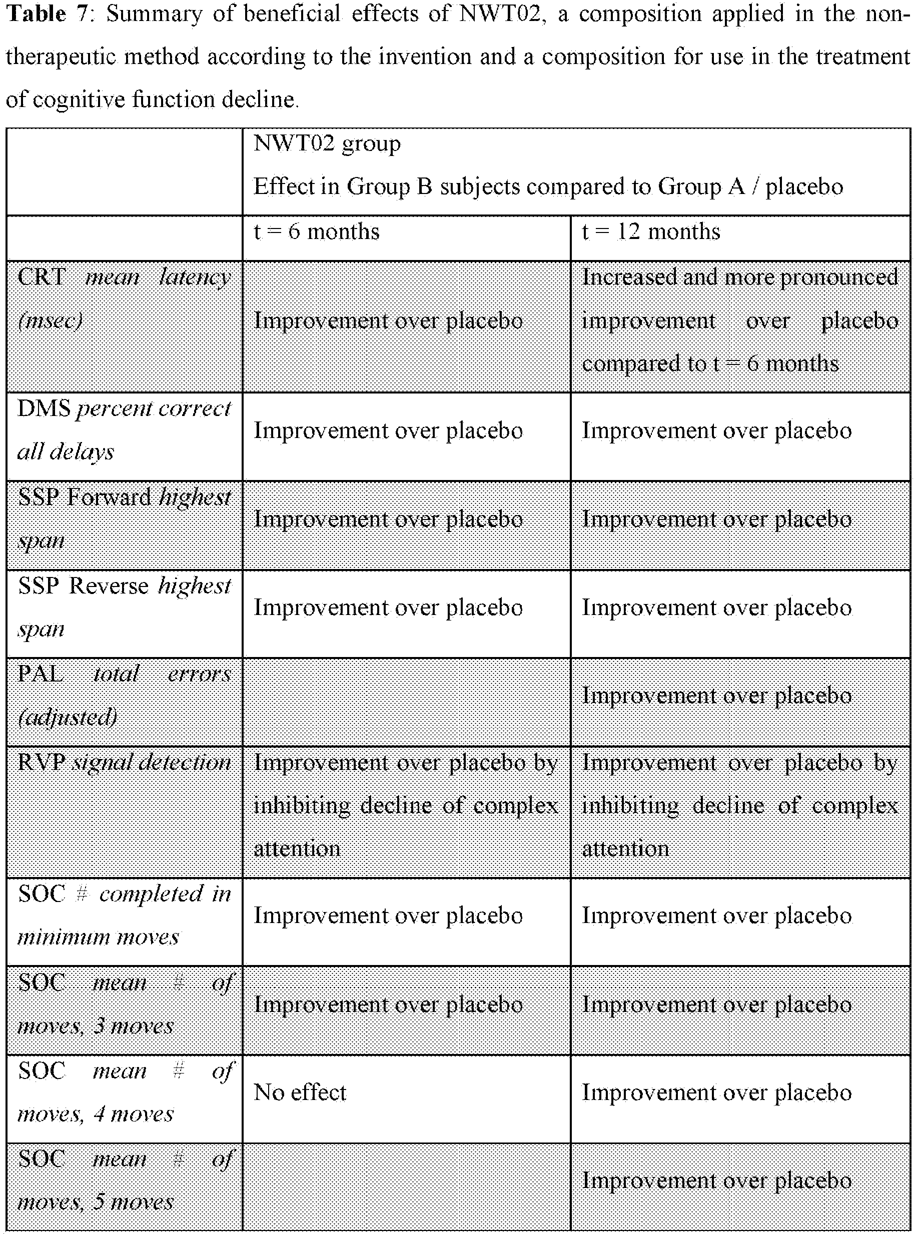

### Example: Subject stratification based on age and treatment group

Subjects who are subjected to the aforementioned cognition tests are divided first based on the treatment (placebo versus administration of a composition of the invention, here referred to as NWT-02; Group A versus Group B), and subsequently further divided based on age at the time of treatment / cognition testing ('Young' when younger than 70 years of age, 'Old' when 70 years of age or older) (See Table 8). This two-stage sub-division of subjects allows for subject stratification relating to an effect of intake of a composition of the invention on the outcome of cognition tests performed, in the context of age of the subject.

Surprisingly, it was found that for subjects younger than 70 years of age, administering a composition of the invention had a particularly pronounced effect on improvement of cognition, compared to the placebo group A of the same age. In particular, subjects younger than 70 years of age and to whom the composition of the invention was administered scored better on at least the DMS test, the SSP forward test, the RVP B test and the 'SOC min problems solved' test, compared to the placebo group of subjects having the same age of 70 years of age or younger.

**Table 8. Subjects Characteristics - younger (<70 years) versus older (>70 years) subjects**

| | *Young (< 70 years)* | | *Old (> 70 years)* | |
|---|---|---|---|---|
| | Group A (n=3) | Group B (n=4) | Group A (n=3) | Group B (n=3) |
| Age | 61.7 ± 5.9 | 64.0 ± 6.2 | 86.3 ± 9.0 | 74.7 ± 3.2 |

### Example: Subject stratification based on AREDS stage of age-related macular degeneration

Subjects who are subjected to the aforementioned cognition tests as outlined in the Examples are divided first based on the treatment (placebo versus administration of a composition of the invention, here referred to as NWT-02; Group A versus Group B), and subsequently further divided based on the AREDS stage of the age-related macular degeneration the subjects where suffering from. This two-stage sub-division of subjects allows for subject stratification relating to an effect of intake of a composition of the invention on the outcome of cognition tests performed, in the context of the degree of age-related macular degeneration of the subject, based on AREDS grading.

Surprisingly, it was found that for subjects having intermediate age-related macular degeneration graded as 3 according to the AREDS scaling or having late AMD at AREDS grade 4, administering the composition of the invention had a particularly pronounced effect on improvement of cognition, compared to the placebo group of the subjects having intermediate AMD and late AMD, AREDS grade 3 and 4. In particular, subjects with intermediate AMD and late AMD, AREDS grade 3 and 4, and to whom the composition 'NWT-02' of the invention was administered, scored better on at least the DMS test, SSPforward test, 'PAL total errors' test and 'SOC min problems solved' test, compared to the placebo group of subjects having the same stage of AMD relating to AREDS grade 3 and 4.

**Table 9. Subject characteristics - subjects that were classified into AREDS grade 1 or 2 (no AMD or early AMD) versus those subjects classified into AREDS grade 3 or 4 (intermediate AMD or late AMD)**

| | AREDS Grade 1, 2 AMD | | AREDS Grade 3, 4 AMD | |
|---|---|---|---|---|
| | Group A (n=1) | Group B (n=3) | Group A (n=5) | Group B (n=4) |
| composition | placebo | NWT-02 | placebo | NWT-02 |

## Claims

1. Non-therapeutic method of amelioration of a cognitive function in a subject, the method comprising administering to the subject a composition comprising:
(1) at least one omega-3 fatty acid; and
(2) lutein and zeaxanthin, wherein the lutein is dosed to provide an amount of between 0,5 mg and 5 mg of lutein per day and the zeaxanthin is dosed to provide an amount of between 0,15 mg and 0,40 mg of zeaxanthin per day.

2. Composition comprising at least one omega-3 fatty acid and lutein and zeaxanthin, for use in the prevention or the treatment of the decline of a cognitive function of a subject or for use in the amelioration of a cognitive function of a subject, wherein the lutein is dosed to provide an amount of between 0,5 mg and 5 mg of lutein per day and the zeaxanthin is dosed to provide an amount of between 0,15 mg and 0,40 mg of zeaxanthin per day.

3. The non-therapeutic method according to claim 1 or the composition for use according to claim 2, wherein the cognitive function of the subject was previously declined at the start of administering the composition to the subject.

4. The non-therapeutic method according to claim 1 or 3 or the composition for use according to any one of the claims 2-3, wherein the at least one omega-3 fatty acid is selected from the group consisting of docosahexaenoic acid and eicosapentaenoic acid, preferably the at least one omega-3 fatty acid is docosahexaenoic acid.

5. The non-therapeutic method according to any one of the claims 1 or 3-4 or the composition for use according to any one of the claims 2-4, wherein the subject is a healthy subject.

6. The non-therapeutic method according to any one of the claims 1 or 3-5 or the composition for use according to any one of the claims 2-5, wherein the subject is a subject with intermediate age-related macular degeneration or a subject with late age-related macular degeneration.

7. The non-therapeutic method according to any one of the claims 1 or 3-6 or the composition for use according to any one of the claims 2-6, wherein the subject is human.

8. The non-therapeutic method according to any one of the claims 1 or 3-7 or the composition for use according to any one of the claims 2-7, wherein the improved cognitive function is any one or more of general alertness, motor speed, short-term memory such as short-term visual recognition memory, working memory, visual memory, new learning, sustained attention, and planning efficiency, preferably the cognitive function is any one or more of the group consisting of learning and memory, complex attention and executive functioning, more preferably the improved cognitive function is at least two of the cognitive functions of said group, most preferably the learning and memory, complex attention and executive functioning are all improved.

9. The non-therapeutic method according to any one of the claims 1 or 3-8 or the composition for use according to any one of the claims 2-8, wherein the subject is over 40 years old, preferably over 50 years old such as between 58 and 70 years of age, more preferably over 60 years old such as about 64 years of age, most preferably over 70 years old.

10. The non-therapeutic method according to any one of the claims 1 or 3-9 or the composition for use according to any one of the claims 2-9, wherein the at least one xanthophyll is contained in an aqueous dispersion.

11. The non-therapeutic method according to claim 10 or the composition for use according to claim 10, wherein the aqueous dispersion is selected from the group consisting of skimmed milk, semi-skimmed milk, buttermilk, a buttermilk fraction, fermented milk, yoghurt, soy drink, soy milk, fermented soy milk, fruit juices, fruit purees, syrups, vegetable juices, vegetable purees.

12. The non-therapeutic method according to claim 10 or the composition for use according to claim 10, wherein the aqueous dispersion is buttermilk or a buttermilk fraction.

13. The non-therapeutic method according to any one of the claims 1 or 3-12 or the composition for use according to any one of the claims 2-12, wherein the composition comprises egg yolk.

14. The non-therapeutic method according to any one of the claims 1 or 3-13 or the composition for use according to any one of the claims 2-13, wherein the composition is dehydrated.

15. The non-therapeutic method according to any one of the claims 1 or 3-14 or the composition for use according to any one of the claims 2-14, wherein the composition is contained in a food product.

16. The non-therapeutic method according to any one of the claims 1 or 3-15 or the composition for use according to any one of the claims 2-15, wherein the composition is in the form of a dietary supplement.

17. The non-therapeutic method according to any one of the claims 1 or 3-16 or the composition for use according to any one of the claims 2-16, wherein the composition is in the form of a foodstuff, such as a drink, that has been enriched with the composition.

18. The non-therapeutic method according to any one of the claims 1 or 3-17 or the composition for use according to any one of the claims 2-17, wherein the at least one omega-3 fatty acid is dosed to provide an amount of between about 50 mg and 3 g of omega-3 fatty acid per day, preferably at least about 60 mg, more preferably at least about 75 mg, most preferably between about 120 mg and 600 mg.

19. The non-therapeutic method according to claim 1 or the composition for use according to claim 2, wherein the lutein is dosed to provide an amount of between about 1,1 mg and 1,8 mg lutein per day, and/or the zeaxanthin is dosed to provide an amount of between about 0,17 mg and 0,30 mg zeaxanthin per day.

20. The non-therapeutic method according to claim 1 or the composition for use according to claim 2, wherein the lutein is dosed to provide an amount of about 1,1 mg lutein per day and the zeaxanthin is dosed to provide an amount of about 0,20 mg zeaxanthin per day.

21. The non-therapeutic method according to any one of the claims 1 or 3-19 or the composition for use according to any one of the claims 2-19, wherein the at least one omega-3 fatty acid is docosahexaenoic acid and is dosed to provide an amount of about 170 mg per day.

## Patentansprüche

1. Nicht-therapeutisches Verfahren zur Verbesserung einer kognitiven Funktion bei einem Subjekt, wobei das Verfahren das Verabreichen einer Zusammensetzung umfassend:
(1) mindestens eine Omega-3-Fettsäure; und
(2) Lutein und Zeaxanthin, wobei das Lutein dosiert ist, um eine Menge zwischen 0,5 mg und 5 mg Lutein pro Tag bereitzustellen, und das Zeaxanthin dosiert ist, um eine Menge zwischen 0,15 mg und 0,40 mg Zeaxanthin pro Tag bereitzustellen,
an das Subjekt umfasst.

2. Zusammensetzung, umfassend mindestens eine Omega-3-Fettsäure und Lutein und Zeaxanthin, zur Anwendung bei der Vorbeugung oder der Behandlung des Rückgangs einer kognitiven Funktion eines Subjekts oder zur Anwendung bei der Verbesserung einer kognitiven Funktion eines Subjekts, wobei das Lutein dosiert ist, um eine Menge zwischen 0,5 mg und 5 mg Lutein pro Tag bereitzustellen, und das Zeaxanthin dosiert ist, um eine Menge zwischen 0,15 mg und 0,40 mg Zeaxanthin pro Tag bereitzustellen.

3. Nicht-therapeutisches Verfahren nach Anspruch 1 oder Zusammensetzung zur Anwendung nach Anspruch 2, wobei die kognitive Funktion des Subjekts zuvor vermindert zu Beginn der Verabreichung der Zusammensetzung an das Subjekt war.

4. Nicht-therapeutisches Verfahren nach Anspruch 1 oder 3 oder Zusammensetzung zur Anwendung nach einem der Ansprüche 2-3, wobei die mindestens eine Omega-3-Fettsäure ausgewählt ist aus der Gruppe bestehend aus Docosahexaensäure und Eicosapentaensäure Säure, vorzugsweise ist die mindestens eine Omega-3-Fettsäure Docosahexaensäure.

5. Nicht-therapeutisches Verfahren nach einem der Ansprüche 1 oder 3-4 oder Zusammensetzung zur Anwendung nach einem der Ansprüche 2-4, wobei das Subjekt ein gesundes Subjekt ist.

6. Nicht-therapeutisches Verfahren nach einem der Ansprüche 1 oder 3-5 oder Zusammensetzung zur Anwendung nach einem der Ansprüche 2-5, wobei das Subjekt ein Subjekt mit mittlerer altersbedingter Makuladegeneration oder ein Subjekt mit später altersbedingter Makuladegeneration ist.

7. Nicht-therapeutisches Verfahren nach einem der Ansprüche 1 oder 3-6 oder Zusammensetzung zur Anwendung nach einem der Ansprüche 2-6, wobei das Subjekt ein Mensch ist.

8. Nicht-therapeutisches Verfahren nach einem der Ansprüche 1 oder 3-7 oder Zusammensetzung zur Anwendung nach einem der Ansprüche 2-7, wobei die verbesserte kognitive Funktion eine oder mehrere von allgemeiner Wachsamkeit, motorischer Geschwindigkeit, Kurzzeitgedächtnis wie kurzfristigem visuellem Erkennungsgedächtnis, Arbeitsgedächtnis, visuellem Gedächtnis, neuem Lernen, anhaltender Aufmerksamkeit und Planungseffizienz ist, vorzugsweise ist die kognitive Funktion eine oder mehrere aus der Gruppe bestehend aus Lernen und Gedächtnis, komplexer Aufmerksamkeit und exekutiver Funktion, stärker bevorzugt ist die verbesserte kognitive Funktion mindestens zwei der kognitiven Funktionen der Gruppe, am meisten bevorzugt sind das Lernen und das Gedächtnis, die komplexe Aufmerksamkeit und die exekutive Funktion alle verbessert.

9. Nicht-therapeutisches Verfahren nach einem der Ansprüche 1 oder 3-8 oder Zusammensetzung zur Anwendung nach einem der Ansprüche 2-8, wobei das Subjekt über 40 Jahre alt ist, vorzugsweise über 50 Jahre alt ist, wie z.B. zwischen 58 und 70 Jahre alt, bevorzugter über 60 Jahre alt, wie etwa 64 Jahre alt, am meisten bevorzugt über 70 Jahre alt.

10. Nicht-therapeutisches Verfahren nach einem der Ansprüche 1 oder 3-9 oder Zusammensetzung zur Anwendung nach einem der Ansprüche 2-9, wobei das mindestens eine Xanthophyll in einer wässrigen Dispersion enthalten ist.

11. Nicht-therapeutisches Verfahren nach Anspruch 10 oder Zusammensetzung zur Anwendung nach Anspruch 10, wobei die wässrige Dispersion ausgewählt ist aus der Gruppe bestehend aus Magermilch, teilentrahmter Milch, Buttermilch, einer Buttermilchfraktion, fermentierter Milch, Joghurt, Sojagetränk, Sojamilch, fermentierter Sojamilch, Fruchtsäften, Fruchtpürees, Sirups, Gemüsesäften, Gemüsepürees.

12. Nicht-therapeutisches Verfahren nach Anspruch 10 oder Zusammensetzung zur Anwendung nach Anspruch 10, wobei die wässrige Dispersion Buttermilch oder eine Buttermilchfraktion ist.

13. Nicht-therapeutisches Verfahren nach einem der Ansprüche 1 oder 3-12 oder Zusammensetzung zur Anwendung nach einem der Ansprüche 2-12, wobei die Zusammensetzung Eigelb umfasst.

14. Nicht-therapeutisches Verfahren nach einem der Ansprüche 1 oder 3-13 oder Zusammensetzung zur Anwendung nach einem der Ansprüche 2-13, wobei die Zusammensetzung entwässert ist.

15. Nichttherapeutisches Verfahren nach einem der Ansprüche 1 oder 3-14 oder Zusammensetzung zur Anwendung nach einem der Ansprüche 2-14, wobei die Zusammensetzung in einem Lebensmittelprodukt enthalten ist.

16. Nicht-therapeutisches Verfahren nach einem der Ansprüche 1 oder 3-15 oder Zusammensetzung zur Anwendung nach einem der Ansprüche 2-15, wobei die Zusammensetzung in Form eines Nahrungsergänzungsmittels vorliegt.

17. Nichttherapeutisches Verfahren nach einem der Ansprüche 1 oder 3-16 oder Zusammensetzung zur Anwendung nach einem der Ansprüche 2-16, wobei die Zusammensetzung in Form eines Lebensmittels, wie eines Getränks, vorliegt, das mit der Zusammensetzung angereichert wurde.

18. Nicht-therapeutisches Verfahren nach einem der Ansprüche 1 oder 3-17 oder Zusammensetzung zur Anwendung nach einem der Ansprüche 2-17, wobei die mindestens eine Omega-3-Fettsäure dosiert wird, um eine Menge von zwischen etwa 50 mg und 3 g Omega-3-Fettsäure pro Tag bereitzustellen, vorzugsweise mindestens etwa 60 mg, stärker bevorzugt mindestens etwa 75 mg, am meisten bevorzugt zwischen etwa 120 mg und 600 mg.

19. Nicht-therapeutisches Verfahren nach Anspruch 1 oder Zusammensetzung zur Anwendung nach Anspruch 2, wobei das Lutein dosiert wird, um eine Menge zwischen etwa 1,1 mg und 1,8 mg Lutein pro Tag bereitzustellen, und/oder das Zeaxanthin dosiert wird, um eine Menge zwischen 0,17 mg und 0,30 mg Zeaxanthin pro Tag bereitzustellen.

20. Nicht-therapeutisches Verfahren nach Anspruch 1 oder Zusammensetzung zur Anwendung nach Anspruch 2, wobei das Lutein dosiert wird, um eine Menge von etwa 1,1 mg Lutein pro Tag bereitzustellen, und das Zeaxanthin dosiert wird, um eine Menge von etwa 0,20 mg Zeaxanthin pro Tag bereitzustellen.

21. Nicht-therapeutisches Verfahren nach einem der Ansprüche 1 oder 3-19 oder Zusammensetzung zur Anwendung nach einem der Ansprüche 2-19, wobei die mindestens eine Omega-3-Fettsäure Docosahexaensäure ist und dosiert wird, um eine Menge von etwa 170 mg pro Tag bereitzustellen.

## Revendications

1. Procédé non thérapeutique d'amélioration d'une fonction cognitive chez un sujet, ledit procédé comprenant l'administration au sujet d'une composition comprenant :
(1) au moins un acide gras oméga 3 ; et
(2) de la lutéine et de la zéaxanthine, où la lutéine est dosée pour apporter une quantité comprise entre 0,5 et 5 mg de lutéine par jour et la zéaxanthine est dosée pour apporter une quantité comprise entre 0,15 mg et 0,40 mg de zéaxanthine par jour.

2. Composition comprenant au moins un acide gras oméga 3 et de la lutéine et de la zéaxanthine, destinée à être utilisée dans la prévention ou le traitement du déclin d'une fonction cognitive d'un sujet ou destinée à être utilisée dans l'amélioration d'une fonction cognitive d'un sujet, dans laquelle la lutéine est dosée pour apporter une quantité comprise entre 0,5 mg et 5 mg de lutéine par jour et la zéaxanthine est dosée pour apporter une quantité comprise entre 0,15 mg et 0,40 mg de zéaxanthine par jour.

3. Procédé non thérapeutique selon la revendication 1 ou composition destinée à être utilisée selon la revendication 2, où la fonction cognitive du sujet avait déjà décliné au début de l'administration de la composition au sujet.

4. Procédé non thérapeutique selon la revendication 1 ou 3, ou composition destinée à être utilisée selon l'une quelconque des revendications 2-3, où ledit au moins un acide gras oméga 3 est choisi dans le groupe constitué par l'acide docosahexaénoïque et l'acide éicosapentaénoïque, de préférence, ledit au moins un acide gras oméga 3 est l'acide docosahexaénoïque.

5. Procédé non thérapeutique selon l'une quelconque des revendications 1 ou 3-4, ou composition destinée à être utilisée selon l'une quelconque des revendications 2-4, où le sujet est un sujet sain.

6. Procédé non thérapeutique selon l'une quelconque des revendications 1 ou 3-5, ou composition destinée à être utilisée selon l'une quelconque des revendications 2-5, où le sujet est un sujet présentant une dégénérescence maculaire liée à l'âge au stade intermédiaire ou un sujet présentant une dégénérescence maculaire tardive liée à l'âge.

7. Procédé non thérapeutique selon l'une quelconque des revendications 1 ou 3-6, ou composition destinée à être utilisée selon l'une quelconque des revendications 2-6, où le sujet est un être humain.

8. Procédé non thérapeutique selon l'une quelconque des revendications 1 ou 3-7, ou composition destinée à être utilisée selon l'une quelconque des revendications 2-7, où la fonction cognitive améliorée est l'une quelconque ou plusieurs fonctions parmi la vigilance générale, la vitesse motrice, la mémoire à court terme telle que la mémoire de reconnaissance visuelle à court terme, la mémoire de travail, la mémoire visuelle, le nouvel apprentissage, l'attention soutenue, et l'efficacité de planification, de préférence, la fonction cognitive est l'une quelconque ou plusieurs fonctions du groupe constitué par l'apprentissage et la mémoire, l'attention complexe et le fonctionnement exécutif, plus préférablement, la fonction cognitive améliorée est au moins deux des fonctions cognitives dudit groupe, le plus préférablement, l'apprentissage et la mémoire, l'attention complexe et le fonctionnement exécutif sont tous améliorés.

9. Procédé non thérapeutique selon l'une quelconque des revendications 1 ou 3-8, ou composition destinée à être utilisée selon l'une quelconque des revendications 2-8, où le sujet est âgé de plus de 40 ans, de préférence, plus de 50 ans comme par exemple entre 58 et 70 ans, plus préférablement, plus de 60 ans, comme par exemple âgé d'environ 64 ans, le plus préférablement, plus de 70 ans.

10. Procédé non thérapeutique selon l'une quelconque des revendications 1 ou 3-9, ou composition destinée à être utilisée selon l'une quelconque des revendications 2-9, où ladite au moins une xanthophylle est contenue dans une dispersion aqueuse.

11. Procédé non thérapeutique selon la revendication 10, ou composition destinée à être utilisée selon la revendication 10, où la dispersion aqueuse est choisie dans le groupe constitué par le lait écrémé, le lait demi-écrémé, le babeurre, une fraction de babeurre, le lait fermenté, le yaourt, les boissons au soja, le lait de soja, le lait de soja fermenté, les jus de fruits, les purées de fruits, les sirops, les jus de légumes, les purées de légumes.

12. Procédé non thérapeutique selon la revendication 10, ou composition destinée à être utilisée selon la revendication 10, où la dispersion aqueuse est le babeurre ou une fraction de babeurre.

13. Procédé non thérapeutique selon l'une quelconque des revendications 1 ou 3-12, ou composition destinée à être utilisée selon l'une quelconque des revendications 2-12, où la composition comprend du jaune d'œuf.

14. Procédé non thérapeutique selon l'une quelconque des revendications 1 ou 3-13, ou composition destinée à être utilisée selon l'une quelconque des revendications 2-13, où la composition est déshydratée.

15. Procédé non thérapeutique selon l'une quelconque des revendications 1 ou 3-14, ou composition destinée à être utilisée selon l'une quelconque des revendications 2-14, où la composition est contenue dans un produit alimentaire.

16. Procédé non thérapeutique selon l'une quelconque des revendications 1 ou 3-15, ou composition destinée à être utilisée selon l'une quelconque des revendications 2-15, où la composition se présente sous forme de complément alimentaire.

17. Procédé non thérapeutique selon l'une quelconque des revendications 1 ou 3-16, ou composition destinée à être utilisée selon l'une quelconque des revendications 2-16, où la composition se présente sous forme d'une denrée alimentaire, telle qu'une boisson, qui a été enrichie avec la composition.

18. Procédé non thérapeutique selon l'une quelconque des revendications 1 ou 3-17, ou composition destinée à être utilisée selon l'une quelconque des revendications 2-17, où ledit au moins un acide gras oméga 3 est dosé pour apporter une quantité comprise entre environ 50 mg et 3 g d'acide gras oméga 3 par jour, de préférence au moins environ 60 mg, plus préférablement au moins environ 75 mg, le plus préférablement entre environ 120 mg et 600 mg.

19. Procédé non thérapeutique selon la revendication 1, ou composition destinée à être utilisée selon la revendication 2, où la lutéine est dosée pour apporter une quantité comprise entre environ 1,1 mg et 1,8 mg de lutéine par jour, et/ou la zéaxanthine est dosée pour apporter une quantité comprise entre environ 0,17 mg et 0,30 mg de zéaxanthine par jour.

20. Procédé non thérapeutique selon la revendication 1, ou composition destinée à être utilisée selon la revendication 2, où la lutéine est dosée pour apporter une quantité d'environ 1,1 mg de lutéine par jour, et la zéaxanthine est dosée pour apporter une quantité d'environ 0,20 mg de zéaxanthine par jour.

21. Procédé non thérapeutique selon l'une quelconque des revendications 1 ou 3-19, ou composition destinée à être utilisée selon l'une quelconque des revendications 2-19, où ledit au moins un acide gras oméga 3 est l'acide docosahexaénoïque et est dosé pour apporter une quantité d'environ 170 mg par jour.
